# EUROPEAN PATENT SPECIFICATION

(11) **EP 3 936 584 B1**
(45) Date of publication and mention of the grant of the patent: **13.08.2025**
(21) Application number: 19919342.6
(22) Date of filing: 08.03.2019
(51) Int. Cl.: C09K 5/16, A61F 7/08

(54) **DISPOSABLE AUTONOMOUS THERMAL DEVICE**
AUTONOME THERMISCHE EINWEGVORRICHTUNG
DISPOSITIF THERMIQUE AUTONOME JETABLE

(43) Date of publication of application: 12.01.2022
(73) Proprietor: Global Termobiomasa S.L., 35600 Las Palmas (ES)
(72) Inventor: DOSAL ARIZA, Eva, 35600 Las Palmas (ES); FERNÁNDEZ GARCÍA, Juan José, 35600 Las Palmas (ES)
(74) Representative: Del Valle Valiente, Sonia
(86) International application number: PCT/ES2019/070156
(87) International publication number: WO 2020/183034

(56) References cited:
- WO-A1-2018/230651
- WO-A2-2010/080898
- CA-A- 1 127 392
- US-A1- 2012 006 314

## Description

### OBJECT OF THE INVENTION

The invention, as stated in the title of the present specification, relates to a disposable autonomous thermal device which contributes, to its intended use, advantages and features, which are described in detail further on, which imply an improvement in the current state of the art.

More particularly, the object of the invention focuses on a disposable autonomous thermal device that provides heat to living beings, garments or objects with very different purposes, for which it comprises a container with a composition that, in contact with a liquid, generates a controlled exothermal reaction, said composition consisting of a mixture of two chemical substances, wherein at least one of said substances is an acid and the other a base, both being wrapped or contained in a container in the form of a film or sheet.

### FIELD OF APPLICATION OF THE INVENTION

The field of application of the present invention falls within the sector of the industry dedicated to the manufacture of thermal devices.

### BACKGROUND OF THE INVENTION

Autonomous devices that generate heat mainly base the action thereof on three types of exothermal reactions:
- First type: Oxidation reactions due to oxygen in the air and in the presence of humidity of certain metals in powder form such as iron for example. In addition, some also contain activated carbon, metal halides, water-absorbent polymers, tackifiers, among others. Said mixture is contained in a bag, generally made of non-woven fabric, and when the seal is broken the air penetrates and oxidises the metal, initiating the reaction.

These devices are glued directly to the skin or any light garment, by means of a glue that is activated when the seal is removed in order for oxygen to enter and the reaction to begin.

This type of reaction is used, generally for thermal therapeutic uses for the whole body, in the so-called heating pads or heat patches or pad, heat packs, thermal blankets, thermal cushions, thermal bandages, etc., and according to the area of use: knee pads, shoulder pads, collars, back, shoulders, groin (for menstrual pains), hands, feet, etc.

Regarding other uses, to heat a seat from the patent ES282288 (U) - 1985-05-01 of EIXO THERMUS ESPANOLA, S.A. must be noted, which introduces the heating pad in a cover made of spongy and breathable material, which "in response to normal movements of the user acts as a bellows" bringing more air for oxidation to occur, maintaining a temperature above 22 °C.

The disadvantages of this first type of device are, among others, that they can be activated prematurely by the air due to the loss of leak-tightness, taking into account that the humidity already comes in the mixture and that the fabrics containing the mixture (generally of the non-woven fabric type) have pores precisely so that air can enter the pad at the time of use.

Another disadvantage is that friction between the particles over time results in the loss of the exothermal potential thereof. There are patents to try to solve problems of this type, such as applying an exothermal coating made up of oxidisable metal powder, polymer latex, polysaccharide and solvent to the substrate described in patent WO2007078558, as an example of this type of device.

Another disadvantage is the small surface area of the body on which the heating pad acts due to its size, generally 9.5 cm x 13 cm. There are patents that join several pads with flexible materials that also make it possible in some cases to adapt to the part of the body to be treated.

Another disadvantage is that in order to be activated by air, they are permeable to liquids and become inactive when they become damp or wet.

Another disadvantage is that the heat generated is exclusively local in the area of the body where it is applied but cannot heat larger surfaces or volumes.

Another disadvantage is that the maximum temperatures reached are far below those necessary for disinfecting, sanitising, acaricidal or insecticidal effects.
- Second type: Combination reactions between an oxide of a metal or metal salts or metals such as calcium oxide (CaO), sodium hydroxide, potassium hydroxide (KOH), calcium chloride (CaCl2) and magnesium chloride (MgCl2). Electrolytic materials (NaCl, KCI, MgCl 2, FeCl 2, NiCl 2) mix to form a heating element, metals such as aluminium, iron or magnesium or the mixtures thereof, to which when water is added, they generate high temperatures and are used to heat food or beverages as in patent WO/2016/186226 PCT/KR2015/005025 called method for preparing exothermal agent.

Among the disadvantages is that over time and due to the hygroscopic and reactive nature of the elements to be reacted by combination, they become prematurely activated, losing their action.

Other combinations such as calcium oxide and phosphoric acid can explode in addition to being eutrophying for ecosystems.

Some, such as Japanese patent No. 62-9151, do not exceed 50 ° which is insufficient to heat food.

Others with several layers are very expensive like US Patent No. 5,205,277.

In general, the use of chemical compounds of this type is delicate and dangerous and they are not exempt from risks because some are caustic or corrosive or the products resulting from the reaction. In many cases, hydrogen gas is generated with a risk of explosion.

In addition, since the foods are included in the containers, they occupy a lot of space and condition the types of food to a small group.
- And third type: Neutralising reactions between an acid and a base: the patent with some similarity that we have found is the Aquecedor quimico sem chamas BR0200224 (A) - 2003-12-02 in which it uses sodium hydroxide and citric acid (the sodium hydroxide separated in a transparent bag and the citric acid contained in an aluminium metal bag and separated from the water in a bag separated by a cutting line. In a first stage, the water reacts with the sodium hydroxide generating heat, which in turn pierces the metallic seal of the aluminium bag of citric acid, which completes the reaction.) A further example of such a system is described in US2012/006314A1.

The objective of the present invention is, therefore, a thermal device that constitutes an improved alternative to the aforementioned within its field of application, the characterising details that distinguish it being included in the final claims that accompany the present description.

### DESCRIPTION OF THE INVENTION

In general terms, the disposable autonomous thermal device, according to claim 1, that the invention proposes, as noted above, comprises a composition that, made up of a mixture of an acidic substance and a basic substance, is incorporated into a container and, upon contact with a liquid, it generates a controlled exothermal reaction that provides heat and allows multiple uses, several systems having been provided for activating the device in order to enable said composition to come into contact with the liquid, there being multiple apparatuses, objects or specific garments in which the device can be implemented for multiple uses.

In an exemplary embodiment, the disposable autonomous thermal device comprises a mixture of two chemical substances, wherein at least one of these is an acid and the other a base, both being wrapped or contained in a film or sheet.

In another exemplary embodiment, the mixture comprises a first substance from the group of bases or hydroxides, one or more hydroxides or a base of any basic salt or a mixture of same, and further comprise a second substance from the group of acids, one or more inorganic or organic acids or an acid of any acidic salt or a mixture of same.

More specifically, the basic substance comprises one or more of the group of bases or hydroxides, one or more hydroxides or a base any basic salt selected from the group consisting of calcium hydroxide, sodium hydroxide, magnesium hydroxide, nickel hydroxide, zinc hydroxide, iron hydroxide, bromine hydroxide, carbon hydroxide, silver hydroxide, boron hydroxide, lithium hydroxide, copper hydroxide, the mixtures and derivatives thereof or combinations thereof.

And, as for the acidic substance, it comprises one or more inorganic or organic acids or an acid of any acidic salt, one or more acidic components selected from the group consisting of citric acid, acetic acid, adipic acid, alginic acid, amygdalic acid, arsenous acid, ascorbic acid, acetylsalicylic acid, isoascorbic acid, benzoic acid, boric acid, metaboric acid, bromic acid, carbonic acid, carbonaceous acid, caproic acid, cyanic and thiocyanic acid, cinnamic acid, chromic and dichromic acids, hydrocinnamic acid, hydrochloric acid, edetic acid, stearic acid, phenoxyacetic acid, phenylacetic acid, phytic acid, phosphoric acid, hypophosphorous acid, orthophosphoric acid, fruit acids or any other natural acid, formic acid, phosphonic acid, fumaric acid, phthalic acid, gallic acid, glutamic acid, glutamic acid hydrochloride, glutaric acid, hydrochloric acid, lactic acid, lauric acid, malic acid, maleic acid, malonic acid, manganic acid, permanganic acid, nitric acid, oleic acid, orthoarsenic acid, oxalic acid, oxoacid, or hydrogen peroxide, palmitic acid, pelargonic acid, potassium acid, selenous acid, silicic acid, solanthic acid, sorbic acid, succinic acid, tannic acid, tartaric acid, valeric acid, isovaleric acid, 4-methylbenzene sulfonic acid, and mandelic acid, periodic acid, oxacids and hydracids, carboxylic acids in general, derivatives of any of them, any inorganic acidic salt, and the mixtures and derivatives thereof or combinations of the same.

To control and modify, according to each use, the speed, duration, temperature and thermal stability of the device, the following can be modified: the percentage ratio of the ingredients, particle size, the form of the mixture (powder, pellets, tablets, etc.), addition of moisture absorbents, different degrees of vacuum in the container, the size and number of containers that make up autonomous thermal devices that can even be joined together to form a single body. With respect to the aqueous liquid, the amount, composition, form of joining with the ingredients and even the number of shakes after mixing can also vary. Likewise, by means of the different apparatuses or elements adapted to each use and the modification of the features thereof such as the thickness and type of insulation, among others.

In any case, the immediate generation of heat is achieved by contacting an aqueous liquid with the mixture of said substances, which is carried out by means of an activator element that can have various embodiments.

Lastly, it should be noted that the use of heat for the different uses thereof is achieved by implementing the device in different apparatuses and objects.

In this sense, it should be noted that the term container of the device, depending on the application for which it is intended, can correspond to a heating pad, heating pillow, heating bag or heating pack, that is, an element similar to a bag, which is suitable for thermal treatment, as well as with a thermal element, heat generating package, pocket heat generating device, heating apparatus, heating element, heater, thermal cushion.

Moreover, as an example of the multiple applications of use that the device can have, the following applications can be highlighted:
- Thermal therapy: as a device that generates heat to apply to any area of the body of people or animals (contractures, anti-inflammatory, analgesic, hypothermia, colic, etc.).
- Thermal vaporisation of substances, molecules or compounds with uses as a device for:
   i) eliminating or repelling mites, microorganisms, insects, ectoparasites and fungi, among others,
   ii) flavouring food and perfuming, deodorising or purifying environments,
   iii) vaporising molecules, substances or compounds used in allergies, nasal and bronchial congestion, sneezing, cough, pharyngitis, hiccups or singultus and snoring, among others.
- Heating food, objects, clothing items and accessories: as a device for heating, isolating from the cold, preventing and eliminating humidity, keeping warm and defrosting, among others.
- Heating objects and surfaces: as a device for disinfection, sanitisation or pasteurisation purposes against microorganisms, mites and insects:
   i) Thermal acaricide: anti-mite pillow, among others. Mites in their different stages and thermal denaturation of some of the allergenic proteins in clothes, beds, etc.
   ii) Insecticide: pediculicide in humans (antiparasitic thermal cap), fleas and ticks in animals or against nests of insects of exotic species.
   iii) Thermal disinfectant of surfaces such as kitchen cutting boards, walls (antifungal), computer keyboards, floors (thermal mop and its own replacement for same), etc.
   ivi) Thermal disinfectant for objects such as combs, hair ties, nit combs, toothbrushes, clothes, hair bands, shoes, brooms, string mops, flat mops, computer or tablet keyboards, electronic devices, etc.
- Electricity production: as a device in order to charge batteries and electrical or electronic devices.

### DESCRIPTION OF THE DRAWINGS

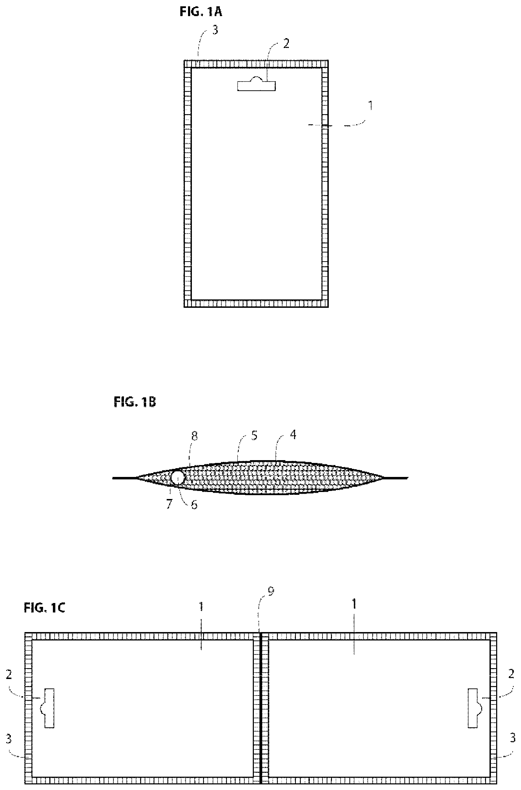

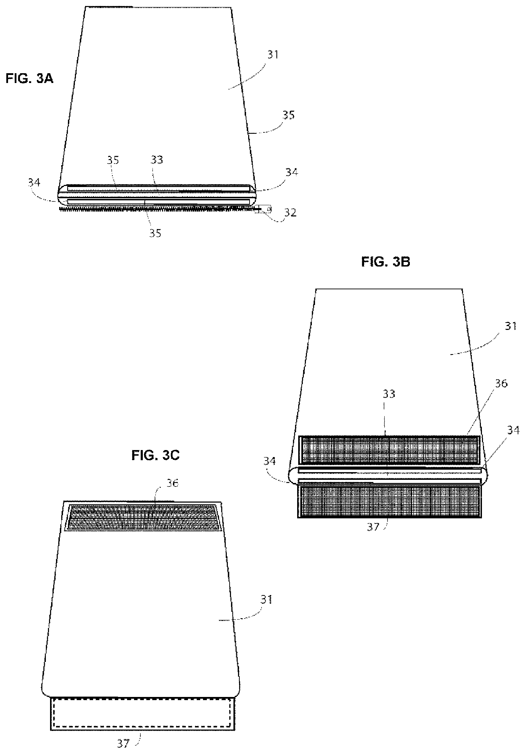

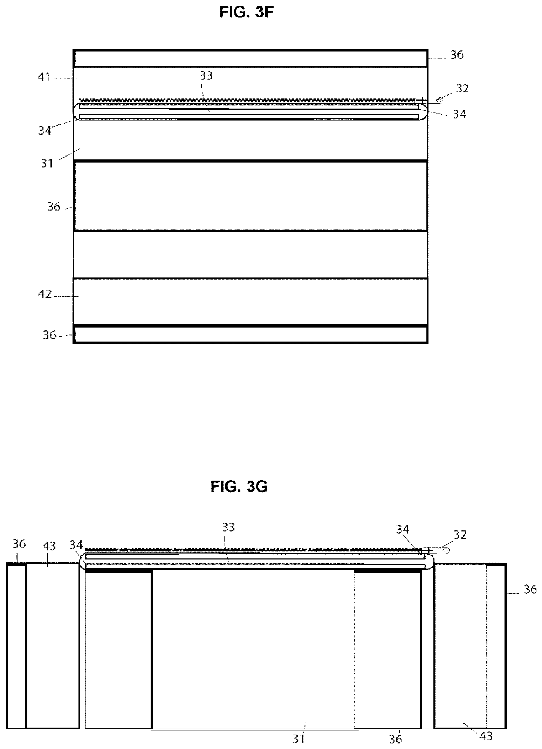

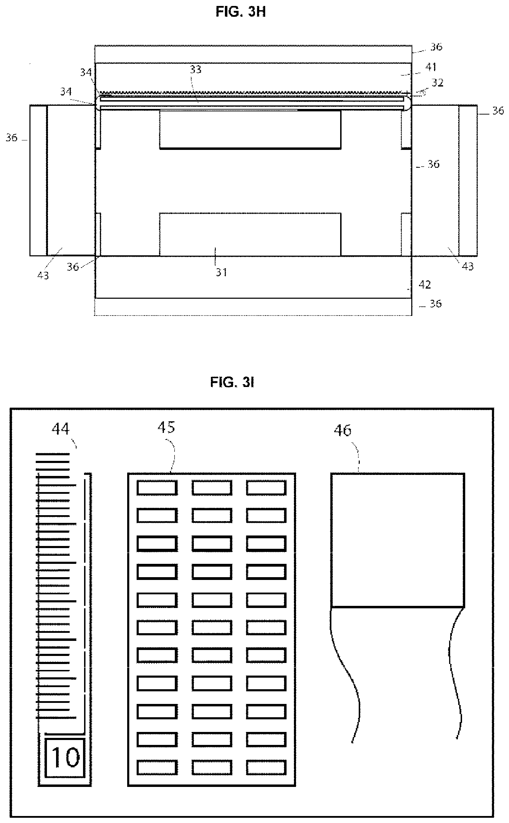

To complement the description provided herein, and for the purpose of helping to make the features of the invention more readily understandable, said description is accompanied by a set of drawings constituting an integral part of the same, which by way of illustration and not limitation represents the following:
Figure number 1 A shows a plan view of a schematic representation of an exemplary embodiment of the disposable autonomous thermal device object of the invention, showing the general external configuration thereof.
Figure number 1B shows a cross-sectional view, according to a cross-sectional cut, of the example of the device, according to the invention, shown in Figure 1A, showing the elements that it comprises internally.
Figure number 1C shows a plan view of another example of the disposable autonomous thermal device of the invention, in this case an example with a double container.
Figures number 2A and 2B show, respectively, a plan view of an example of the heat shield comprising the device according to the invention, and a cross-sectional view of said shield incorporated on the container of the device.
Figure number 3A shows a schematic perspective view of an example of the thermal cover that the device comprises.
Figures number 3B, 3C, 3D and 3E show perspective views of the cover shown in Figure 3A, in respective options for the embodiment thereof with velcro, roll-up, with a pocket, and with magnets.
Figures number 3F, 3G and 3H show respective plan views of the cover with upper and lower tabs, with lateral tabs and with upper and lower and lateral tabs.
Figure number 3I shows a schematic representation of the cover with accessory accessories.
Figures number 4A, 4B, 4C and 4D show respective top and bottom plan and perspective views of the lid and perspective view of the base of an example of an activator of the device, specifically an example to activate it with the thermal cover shown in Figures 3A to 3I.
Figures number 4E and 4F show respective perspective views of the lid and of the base of the activator shown in Figures 4A to 4D, once incorporated into the thermal cover.
Figure 5A shows a schematic perspective view of a harness, as an example of elements that incorporates the thermal device of the invention.
Figures number 5B, 5C, 5D, 5E and 5F show respective views of the harness shown in Figure 5A incorporating the device in different positions and numbers.
Figure number 5G shows a plan view of the belt of the harness shown in the preceding figures.

And Figures 6A and 6B show respective perspective views of the lower portion of a flat mop and of the lid of said flat mop, respectively, as an example of an applicable object to incorporate the thermal device of the invention.

### PREFERRED EMBODIMENT OF THE INVENTION

In view of the aforementioned figures, and in accordance with the numbering adopted, a non-limiting example of the recommended disposable autonomous thermal device can be seen, which comprises the parts and elements indicated and described in detail below.

Considering Figures 1A and 1B, it can be seen how the device in question essentially comprises:
- a body in the form of a container (1) comprising one or more sheets (5);
- an exothermal composition (4) incorporated into the body of the container (1), wherein the exothermal composition (4) consists of the mixture of the acidic substance and the basic substance, which can also contain moisture-absorbing materials;
- and an aqueous liquid (6) separated from the exothermal composition (4), inside or outside the container (1),
wherein, when the exothermal composition (4) of the container (1) is combined with the aqueous liquid (6), either manually or by means of an activating element (50) preferably provided as an auxiliary element of the device, an exothermal neutralising reaction is produced.

The exothermal composition (4), although in a generic way, comprises at least:
- from 1% to 90% by weight of one or more inorganic or organic acids, an acid of any acidic salt or a mixture of same;
- from 1% to 90% by weight of one or more inorganic bases selected from one or more hydroxides or a base of any basic salt or a mixture of same;
- 1% to 90% by weight of one or more moisture-absorbing materials, it contains:
- between 20% and 50% of citric acid;
-
- between 35% and 60% of calcium hydroxide;
- and any absorbent material or materials from among particulate wood, pelletised wood, sodium polyacrylate, cellulose, starches, corn starch, potato starch, katakuri starch, collagen, agar, carrageenan, gelatin, sodium pyrophosphate, furcelleran, polysaccharides, pectin, proteins, amylum, sago, locust bean gum, xanthan gum, guar gum, vegetable gums, arrowroot, tapioca, alginine and mixtures thereof.

It should be noted that, the exothermal composition (4) comprises from 1% to 40% by weight of moisture-absorbing materials, preferably between 10% by weight and 30% by weight of particulate wood.

Said particulate wood of the exothermal composition (4) can be from silvicultural, agricultural, agro-industrial, energy or forestry species selected from the group consisting of olive pits, nut shells, fir, birch, acacia, aspen, carob, cork oak, larch, almond tree, bamboo, cedar, cypress, poplar, juniper, holm oak, eucalyptus, flamboyant, ash, fruit trees, beech, pine, walnut, elm, olive, oak, teak, among others, and mixtures thereof.

In any case, the particulate wood preferably comprises from 1% to 20% by weight of moisture, preferably from 8% to 10% by weight of moisture.

Furthermore, the exothermal composition (4), optionally, has a solid consistency, and can be in the form of a powder, granules, pellets or tablets, preferably in the form of a powder.

Likewise, in an embodiment option, the ingredients of the exothermal composition (4) may be unmixed and separated from each other inside the container (1) of the device, some of which may be loose inside said container (1) and the others inside another container or inner bag of water-soluble material (not represented in the figures) as this, in turn, is inside the main container (1) of the device.

Optionally, some of the ingredients of the exothermal composition (4), not mixed and separated in the container (1) of the device, can be inside a first container or inner bag of water-soluble material and the others inside a second container or other inner bag of water-soluble material, that is, both containers or both bags being water-soluble inside the main container (1) of the device.

Alternatively, however, the ingredients of the exothermal composition (4) may be mixed and bonded inside the device container (1), all of which may be loose inside the main container (1) of the device or inside an inner bag of water-soluble material in turn incorporated inside said container (1) of the device.

As for the aqueous liquid (6) comprised by the container (1) of the device, it is selected from the group consisting of water, distilled water, double distilled water, deionised water, mineral water, spring water, water with salts, brackish water, salt water, or any of the mixtures thereof, preferably distilled water.

In any case, said aqueous liquid (6) is added to the exothermal composition (4) from 5 to 80 parts by weight of this composition, preferably from 20 to 30 parts by weight, in order to generate heat by a neutralising reaction.

The aqueous liquid (6) is a solution with the acid, a solution with one or more bases or a solution with hydrogen peroxide or alcohol, preferably a solution with hydrogen peroxide, wherein the hydrogen peroxide of said solution preferably comprises from 0.20% by weight to 10% of the solution, and preferably from 0.50% by weight to 3% by weight of the solution.

More specifically, the hydrogen peroxide of the solution of the aqueous liquid (6) contains a concentration of 1.5% (5 volumes) to a concentration of 50% (200 volumes), preferably between 3% (10 volumes) and 9% (45 volumes).

As has been stated, the aqueous liquid (6) is separated from the exothermal composition (4) that contains the main container (1) of the device, for which, preferably, it is contained in a secondary container (7), it being possible for this secondary container (7) to be inside or outside the container (1) of the device, being the preferred one that is inside the same.

In any case, the secondary container (7) is selected from the group consisting of a vial, bottle, canister, ampoule, glass, bag, terrine and syringe, being in any case preferably a breakable bag or a breakable receptacle.

As previously stated, the aqueous liquid (6) is brought into contact with the exothermal composition (4) or manually, preferably by manual compression in an area (8) of the container (1) of the device to break the secondary container (7), or through an activating element (50) that acts by different means selected from the group comprising injection, breaking of the secondary container (7) by twisting or by mechanical compression.

Preferably, the secondary container (7) where the aqueous liquid (6) is located comprises one or more sheets of polymeric material sealed forming a flexible bag and the materials of which are selected from the group consisting of EVOH, LDPE, MDPE, HDPE, PT/PE, Paper/AL, Paper/PE, PET/AL, PET/PE, PVC/PE, PA/PE, NY/PVDC, PE/PVDC, PP/PVDC, MOPP/PE, Paper/AL/PE, PET/AL/PE, PET/AL/PE, PET/PA/PE, BOPP/AL/PE, BOPP/PE/OPP, BOPP/VMPET/PE, PET/VMPET/PE, PET/AL/CPP, MOPP/Kraft paper/CPP, PET/AL/Paper, PET/PVDC/PE, PET/PT/PE, PT/AL/PE, Wax/Paper/PE, OP/AL/HSL, BOPP/PET/AL/PE, MOPP/PET/AL/PE, Paper/AL/Paper/PE, PT/PE/BOPP/PE, PVDC/PT/PVDC/PE, PVDC/PT/PE/AL/PE, PE/paper/PE/AL/PE/PE, nylon, other laminated coextruded polymeric materials and the combinations thereof, preferably LDPE.

Preferably, the thickness of the polymeric material of one or more sheets of the secondary container (7) containing the aqueous liquid (6) is between 20 and 150 microns, preferably between 50 and 80 microns.

In addition, also preferably, one or more sheets of the secondary container (7) containing the aqueous liquid (6) are hermetically heat-sealed on the perimeter thereof by means of a manual, semi-automatic or automatic sealing or packaging machine, by closing with 4 welds, 3 welds, 3 fillet welds, side folds, with square bottom or Stand-Up, with rear fold, preferably by means of automatic heat closure with 4 welds.

And, alternatively, one or more sheets of polymeric material of the secondary container (7) containing the aqueous liquid (6) are hermetically sealed forming a closed bag.

In addition, the secondary container (7) containing the aqueous liquid (6) can be of different sizes, logically depending on the size of the main container (1) of the device, although preferably it is 8 cm wide x 6 cm long.

As for said container (1) of the device, it comprises one or more sheets of polymeric material sealed forming a flexible bag adaptable to the shape of any part of the human body, animal or object to be heated.

Optionally, the main container (1) is of a water-soluble composition selected from the group consisting of Polyvinyl Alcohol (PVA/PVAL/POVH) and derivatives.

And, when said container (1) is not water-soluble, one or more materials of the composition of the same are selected from the group consisting of EVOH, LDPE, MDPE, HDPE, PT/PE, Paper/AL, Paper/PE, PET/AL, PET/PE, PVC/PE, PA/PE, NY/PVDC, PE/PVDC, PP/PVDC, MOPP/PE, Paper/AL/PE, PET/AL/PE, PET/AL/PE, PET/PA/PE, BOPP/AL/PE, BOPP/PE/OPP, BOPP/VMPET/PE, PET/VMPET/PE, PET/AL/CPP, MOPP/Kraft paper/CPP, PET/AL/Paper, PET/PVDC/PE, PET/PT/PE, PT/AL/PE, wax/paper/PE, OP/AL/HSL, BOPP/PET/AL/PE, MOPP/PET/AL/PE, Paper/AL/Paper/PE, PT/PE/BOPP/PE, PVDC/PT/PVDC/PE, PVDC/PT/PE/AL/PE, PE/paper/PE/AL/PE/PE, nylon, other laminated co-extruded polymeric materials and the combinations thereof.

Optionally, said the non-water-soluble polymeric material of one or more sheets (5) of the main container (1) of the device is preferably Polyester (PET) - Aluminium (AL) - Polyethylene (PE) and, the thickness of said non-water-soluble polymeric material of one or more sheets of the main container (1) of the device is between 20 and 150 microns, preferably between 80 and 100 microns.

In any case, in an embodiment option, one or more sheets (5) of the main container (1) of the device are sealed inside same enabling subdivisions.

Likewise, in another embodiment option, the main container (1) of the device comprises an envelope or wrapper, a vacuum bag or one or more sheets of air bubbles, although this detail has not been represented in the figures.

Also optionally, the container (1) of the device comprises a closure system on one or more of the sides thereof, for example of the easy open type, self-closing, pressure, grip type pressure, zip type, zipper type or slider, velcro, brackets, adhesive type, funnel-type cap, heat-sealed, preferably perimeter heat-sealed.

For this option, and in the same way that has been described for the secondary container (7), one or more sheets (5) of the main container (1) are hermetically heat-sealed on the perimeter edges (3) thereof by means of a manual, semiautomatic or automatic, preferably automatic, sealing or packaging machine by closing with 4 welds, 3 welds, 3 fillet welds, side folds, with square bottom or Stand-Up, preferably by means of automatic hermetic heat closure with 4 welds and with a Euro-Hole type perforation (2).

And, optionally, one or more sheets (5) of polymeric material of the container (1) of the device or main container are hermetically sealed forming a closed bag with different degrees of vacuum, preferably with a high degree of vacuum.

Furthermore, in an embodiment option, the container (1) of the device has adhered to the outside, or in the form of a non-disposable or disposable cover, support or wrapping, preferably made of non-woven fabric, which comprises, or which can be incorporated therein, pharmaceutical compounds, insecticides, essential oils and non-active and active aromatic compounds, extracts and natural plant compounds such as tea oils, neem oil, essential lemon eucalyptus oil, citronella oil, savory, garlic, basil, bergamot, cinnamon, cardamom, onion, cedar, coriander, citronella, clove, juniper, tarragon, eucalyptus, geranium, lemongrass, fennel, bay leaf, lavender, lemon, marjoram, lemon balm, mint, mustard, orange, oregano, rosewood, paprika, parsley, pine, grapefruit, rosemary, sage, soy, thyme, vanilla, etc. or mixtures thereof in order to increase the effectiveness of therapeutic uses, thermal sanitisation, to flavour, insecticide or insect repellent.

Optionally, the container (1) of the device has means of fastening to parts of the body or objects, such as velcro, adhesive, straps, tapes and belts, among others.

Regarding Figure 1C, it is observed how, optionally, the container (1) of the described autonomous thermal device can be joined to the container (1) of other identical or similar autonomous thermal devices by sealing any of the edges (3) thereof, preferably at a heat-sealed seam (9), forming a single body of several of the same.

Furthermore, as mentioned, in order to cause the combination of the exothermal composition (4) of the container (1) with the aqueous liquid (6), in addition to doing so manually, the device contemplates the use of an activator element (50) that may have different configurations, for example in the form of a needle, a clamp, a striker, a threaded push button or others, which can be used either directly on the container (1) of the thermal device or through additional elements incorporated on said container (1) or in which it is housed, such as a heat shield (10) or a thermal cover (31) that are described in detail below.

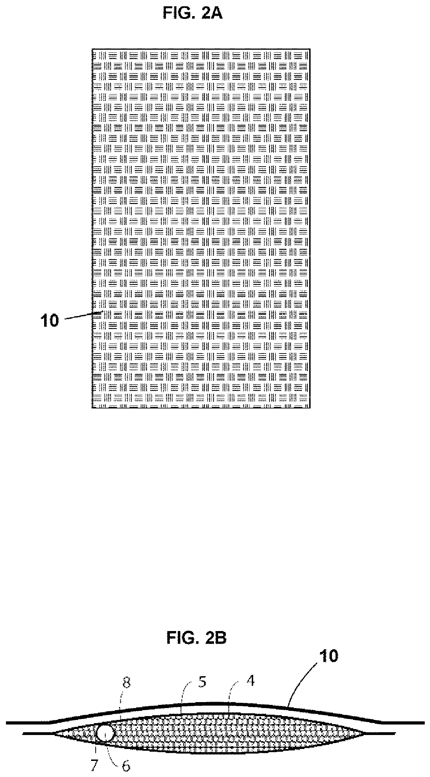

Regarding Figures 2A and 2B, it is observed how the thermal device contemplates the existence of a heat shield (10) the purpose of which is to insulate from the cold and distribute the heat generated by the container (1) of said device, for which such shield (10) preferably comprises one or more layers of bonded superimposed materials and fabrics.

More specifically and preferably, one or more materials of the layers of said heat shield (10) are selected from the group consisting of: wool, polyester, metallic materials such as silver or copper, aerogel, neoprene, wool wadding, acetate, polyester or cotton, among others and any of the mixtures thereof, preferably three layers of neoprene, aerogel and wool.

Preferably, one or more metallic materials of some of the layers of the heat shield (10) are silver or copper, in percentages of 5 to 100%, preferably in percentages of these metals between 5 and 40%, for one or the 2 faces.

Preferably, one or more superimposed layers of the heat shield (10) are joined by any joining system, selected from the group consisting of: stitching, buttons, clasps, zip, velcro, laces and eyelets, studs, glue, hot-melt spray, glue, heat sealing, among others, depending on the object or garment to which it is adhered.

Furthermore, said heat shield (10) can be incorporated and coupled to the device in any non-textile or textile object, item of clothing for people or animals already marketed, such as: pockets, bags, covers for tablets, mobiles and other electronic devices, computers or tablets keyboards, rigid and flexible containers, baby bottle warmers, beverage container warmers, warmers for plates, cutlery, glasses and food, food containers, mops, tiles, rugs, mats, mattresses, air mattresses, beds, bed covers, pillowcases, cots, puffs, seats, chairs, easy chairs, armchairs, cushions, protective seat covers, laundry baskets, tents, sleeping bags for babies, sleeping bags, rucksacks, stuffed animals, toy dolls, hats, caps, neck pads, pillows, anti-mite pillows, scarves, collars, capes, spinal tapes, shoulder pads, harnesses, girdles, bum bags, lumbar pads, elbow pads, mittens, gloves, shoe insoles, socks, hand and foot warmers, trousers, knee braces, ankle braces, boots, pyjamas, dressing gowns, towels, bathrobes, blankets, quilts, vests, life jackets, surf life jackets, bulletproof vests, tactical vests, reflective vests, hunting vests, baseball jackets, raincoats, diving suits, divers suits, pilot suits, astronaut suits, ski suits, cat and dog clothes, cat and dog beds, cushion-pillows for dogs and cats, kennels for dogs and cats, cave-beds for dogs and cats, carriers for dogs and cats, rugs for horses, ovens, vibrators, sex dolls, walls, robot vacuum cleaners and mop robots, among others.

In any case, the heat shield (10) can be located on the objects and items of clothing in the areas near the thermal device and, preferably, covering or located on the container (1) itself of the device. In addition, it can be manufactured in the shape of objects or items of clothing such as a scarf, neck brace, shoulder pads, elbow pads, belt, harnesses, gloves, foot insoles, sleeves, vests, girdles, bum bags, blankets, knee braces, trousers, ankle braces, shoe insoles, socks, towels, among others.

Regarding Figures 3A to 3I, it is observed that the device additionally contemplates the existence of a cover (31), specifically a thermal cover in order to distribute the heat generated by the described thermal device to any object or parts thereof, surfaces, living beings, food or beverages. In addition, depending on the option of carrying out the same, it can be manufactured for purposes such as: heating, conserving heat, drying and defrosting, among others and, optionally, for the purpose of thermal sanitisation of any object, living beings or parts of any thereof, surfaces, food or beverages in order to eliminate mites, microorganisms, insects, fungi and ectoparasites, or for therapeutic uses based on heat on people and animals such as: anti-inflammatory and analgesic for any part of the body including injuries to muscles, ligaments and tendons, menstrual pain, hypothermia, pharyngitis, snoring and contractures, or to vaporise molecules, substances or compounds in order to eliminate or repel mites, microorganisms, insects, ectoparasites and fungi, or to vaporise substances or compounds used in allergies, nasal and bronchial congestion, sneezing, cough, pharyngitis, hiccups or singultus and snoring, or for the purpose of aromatising, perfuming, deodorising or purifying environments. Likewise, a possible use of the cover (31), in one of its embodiment options, is suitable for use thereof to produce electricity in order to charge batteries and electrical or electronic devices.

For this, said cover (31) comprises one or more insulating sheets (34) between which a hollow (33) is defined that houses the container (1) of the autonomous thermal device and a closure system.

Preferably, said one or more sheets (34) are made of one or more types of materials selected from the group comprising polymers of inorganic, natural organic and synthetic organic origin.

Optionally, one or more sheets (34) are made of materials selected from the group consisting of polyurethane or polyester or PVC or polyethylene foam; in foams or agglomerate or extruded or in memory foam; elastomers such as neoprene, lycra or spandex; cork, cardboard, rubber, aerogel, or they consist of a fabric or mesh (35) that incorporates metallic materials such as silver or copper, in percentages of 5 to 100%, preferably in percentages of these metals between 5 and 40%, or mixtures thereof.

In the preferred embodiment, the cover (31) has one or two polyurethane foam sheets (34), in this second case being separated leaving a hollow (33) that houses the autonomous thermal device.

Preferably said polyurethane foam sheets (34) are of a thickness between 1 mm and 300 mm, preferably two sheets, it being possible for the thicknesses of both sheets to be the same or different, preferably both of the same thickness of between 5 and 12 mm.

Preferably, one or both polyurethane foam sheets (34) has (have) a density between 5 kg/m3 and 70 kg/m3, preferably between 10 kg/m3 and 30 kg/m3.

Preferably, one or both of the polyurethane foam sheets (34) are hard or soft, preferably soft and very flexible, which allows it to adapt to any shape depending on the use thereof.

Optionally, the cover (31) is covered on the inside thereof, outside thereof or both by a fabric or a mesh (35).

Preferably, one or more materials of the fabric or mesh (35) that cover it are selected from the group consisting of any natural textile material such as cotton, wool, silk, leather, micro-wool and linen among others, or synthetics such as polyamide, nylon, polyester, PVC, trilaminate, tetralaminate, Gore-tex^{®}, vinyl, neoprene, PU, lycra or spandex, copper or silver metallic fabrics, silicone, latex, neoprene, anti-mite fabric, polyester, aerogel, non-woven fabric, aluminised plastic or not, nylon, vinyl among others or any of the mixtures thereof.

Optionally, the cover (31) contains, on said fabric or mesh (35), an additional covering selected from the group consisting of any textile material, preferably neoprene.

In any case, preferably, the fabric or mesh (35) with which the cover (31) is covered inside, outside or both contains metallic materials such as silver or copper, in percentages from 5 to 100%, preferably in percentages of these metals between 5 and 40%.

In one embodiment option, the arrangement of the sheets or layers of insulating material (34) and fabric (35) is preferably: a sheet of foam rubber covered by fabric on both faces, a hollow (33) that houses the container (1) of the autonomous thermal device and a sheet of aerogel covered by fabric on both faces.

One or more sheets can be made of foamed fabric comprising the fabric and the polyurethane foam sheet.

In any case, the cover (31) can be of any size, according to all the possible and multiple uses as well as according to the number of thermal devices that it incorporates, preferably a size of 150 mm x 250 mm for each thermal device.

In addition, it can have different shapes such as square, rectangular, semi-circular-straight and round, among others.

Likewise, the cover (31) can be single-use or reusable, preferably washable and reusable multiple times.

Furthermore, as a closure system, the cover (31) preferably comprises a zip (32) (Figure 3A), velcro (36), a flap with velcro (37) (Figure 3B), magnets, clasps, snaps or others to insulate inside the thermal device.

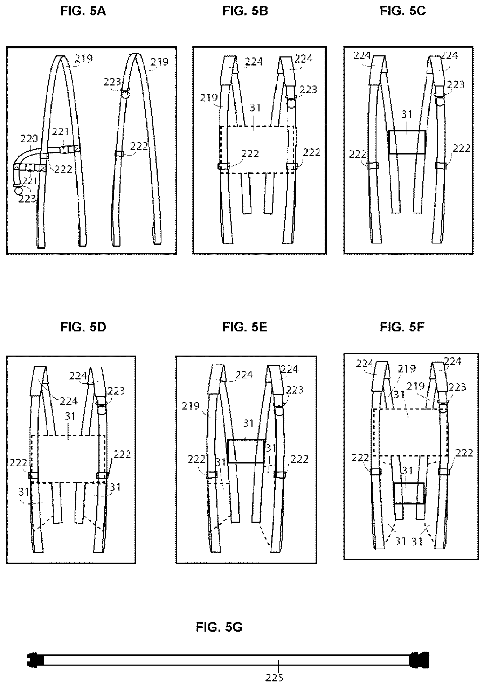

In addition, as can be seen in Figures 3F to 3H, the cover (31) has a configuration that comprises one or more tabs in the upper (41), lower (42) and lateral (43) position, with closure systems, preferably velcro and that serve to adhere and adjust to certain objects such as the straps (219) of a harness as shown in Figures 5A to 5G, or to parts of the body.

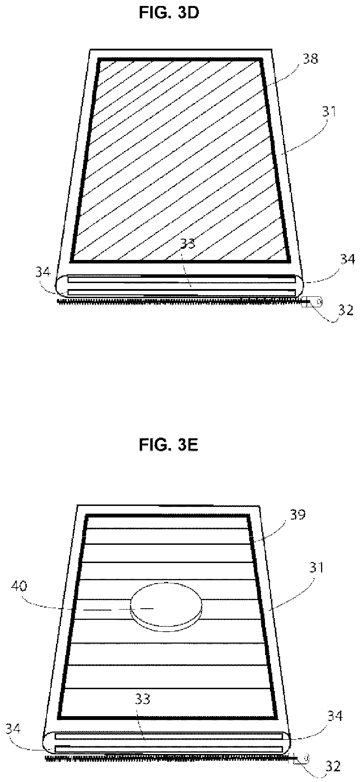

Optionally, it comprises other types of fastening systems to fasten or adapt to the body of people, animals, items of clothing, bedding, sleeping bags, harnesses and any object to be heated, selected from the group consisting of straps, tapes, belts, velcro, glue, adhesive, or for example a magnetic sheet (39) or magnets (40) as shown in Figure 3E, or with buckles, stitching, etc.

In the aforementioned Figure 3E, it is observed how the cover (31), in one embodiment option, comprises a magnetic sheet (39) or a magnet (40) on the outside or inside thereof to fix or adapt to the body of people, animals, items of clothing, bedding, sleeping bags, harnesses and any object to be heated when it comes into contact with another magnetic sheet or other magnet on the external face of the object or garment to which it is fastened.

Optionally, the cover (31) can also comprise an anti-radiation pocket (38), represented in Figure 3D, made of any of the current commercial materials, preferably sewn thereto, to prevent radiation from mobile devices and theft of data of same or of bank cards, etc.

Optionally, the cover (31) contains the heat shield (10) described above, as well as other complementary elements or devices, for example, as shown in Figure 3I, it contains a temperature indicator (44) and/or contains one or more photovoltaic electrical generators (45) to generate and supply electrical energy to charge any battery or directly any electronic mobile device such as mobile phones, computers or tablets among others, or any electrical or electronic apparatus.

Optionally, it can also contain one or more thermoelectric generators (46), preferably one or more Peltier plates that use the heat generated by the thermal device, the container (1) of which is incorporated in the cover (31), by means of the Seebeck effect to generate and supply electrical energy to charge any battery or directly any electronic mobile device such as mobile phones, computers or tablets among others, or any electrical or electronic apparatus.

Preferably, although it has not been represented, it also comprises one or more terminals for charging electronic devices and/or one or more batteries.

Optionally, instead of the container (1) as indicated in the previous paragraphs, the cover (31) is the one that contains a non-disposable or disposable wrapping that comprises, or which can be incorporated therein, pharmaceutical compounds, insecticides, essential oils and non-active and active aromatic compounds, extracts and natural plant compounds such as tea oils, neem oil, essential lemon eucalyptus oil, citronella oil, savory, garlic, basil, bergamot, cinnamon, cardamom, onion, cedar, coriander, citronella, clove, juniper, tarragon, eucalyptus, geranium, lemongrass, fennel, bay leaf, lavender, lemon, marjoram, lemon balm, mint, mustard, orange, oregano, rosewood, paprika, parsley, pine, grapefruit, rosemary, sage, soy, thyme, vanilla, etc. or mixtures thereof.

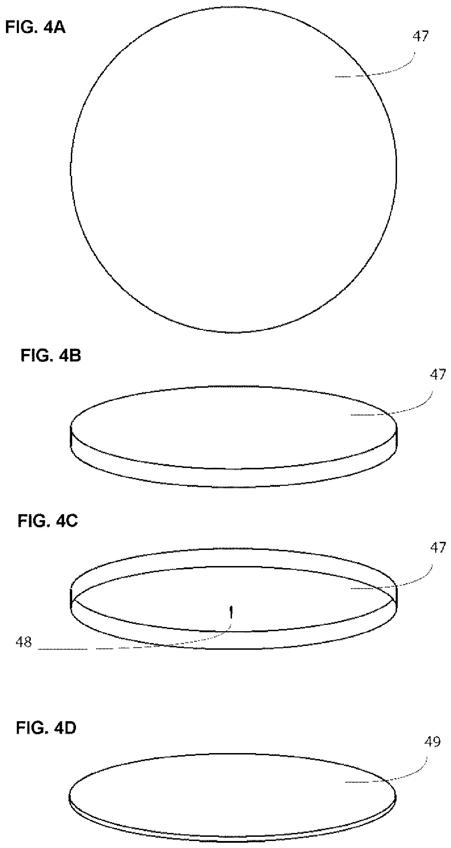

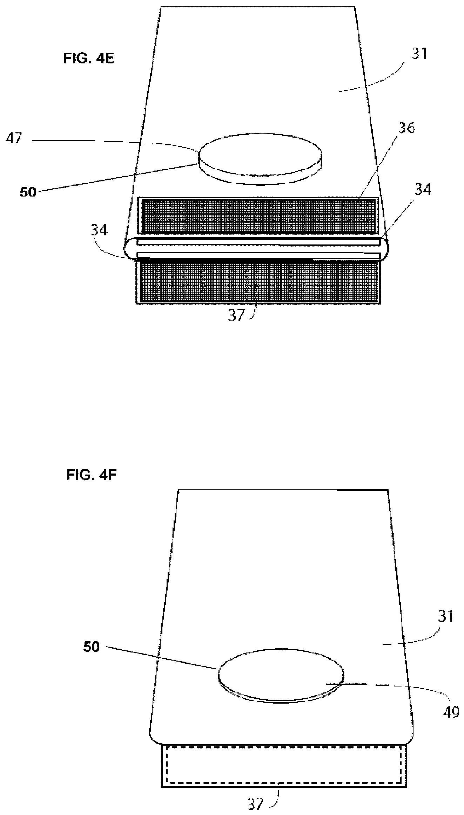

Furthermore, as indicated in previous paragraphs, the device optionally comprises the inclusion of activator elements (50) of the device, applicable directly on the container (1) thereof, or through accessory elements. In this sense, as shown in Figures 4A to 4F, in an embodiment option the invention comprises an activator (50) applicable on the cover (31) described, which comprises a lid (47) with one or more strikers (48) and a base (49), both joined on the outside of the opposite faces of the thermal cover (31), in such a way that, when the lid (47) is pressed, the striker or strikers (48) pass through the thermal cover (31), the sheet (5) of the container (1) of the thermal device and the secondary container (7), and the aqueous liquid (6) comes out contacting the reagents of the exothermal composition (4) activating the exothermal reaction.

In any case, the cover (31) can be manufactured in the shape of any part of the body of people or animals in order to be directly coupled to same.

Likewise, it can be manufactured in the shape of objects or items of clothing such as a scarf, neck brace, shoulder pads, elbow pads, belt, harnesses, gloves, foot insoles, sleeves, vests, girdles, bum bags, blankets, knee braces, trousers, ankle braces, shoe insoles, socks, towels, among others.

In the same way, it can be incorporated and attached to any non-textile or textile object, item of clothing for people or animals.

Thus, as can be seen in Figures 5A to 5G, in a possible application option of the device of the invention, the container (1) can be incorporated, housed in the cover (31) and including or not the heat shield (10), to the straps (219) of a harness to attach it to the body of a user.

More specifically, said harness can be one that has two straps (219) that extend from the front portion of the body to the rear portion, passing over the shoulders, crossing at the neck or not, preferably not crossed, and incorporating one or more thermal covers (31) that house the container (1) of the autonomous thermal device of the invention, for which said cover or covers have fastening systems to fasten or adapt to the harness, such as velcro, glue, adhesive, snaps, zip, sewn, wings or extensions, etc., on two or the four sides thereof, preferably forming part of the body of the cover and sewn to the straps and in such a way that they have different positions and at different heights thanks to the mobility thereof along the straps of the harness, preferably front, rear, right or left lateral positions or any combination thereof, and chest, abdominal, back areas, lumbar region and hips and at any height.

Optionally, the harness has a third strap (220) that is fastened to one of the right or left straps (219) by means of fastening and adjustment systems at both ends, such as carabiners, rings, velcro or buckles, preferably by adjustable buckles (221). Furthermore, preferably, each of the two straps is fitted to a belt (225) by means of an adjustable adjustment buckle (222).

Optionally, both the two straps (219) of the harness and one or more thermal covers (31) contain accessories such as material-holder rings (223), padded protectors (224), pockets, carabiners and pins.

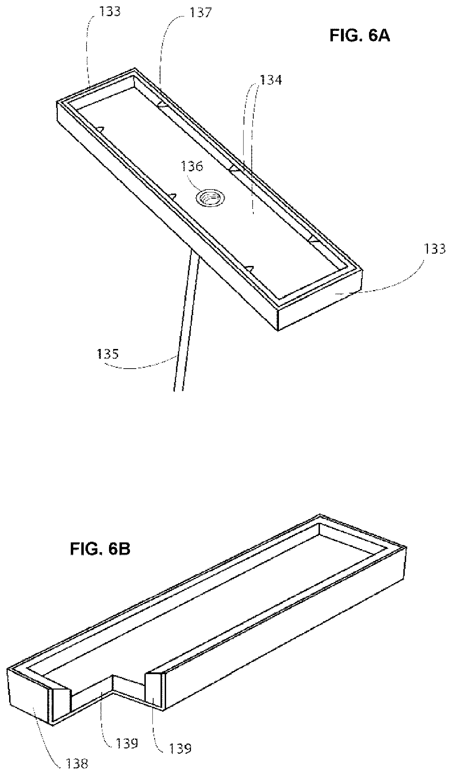

Finally, as can be seen in Figures 6A and 6B, the thermal device of the invention, in a non-limiting embodiment option, for the application thereof housed in an object, is incorporated in a mop that comprises a body (133) provided with a hollow to house the container (1) of said device with a closing lid (138), turning it into a thermal mop.

Preferably, the material of the body and cover of said thermal mop is selected from the group consisting of gold, silver, platinum, copper, aluminium, iron, lead, steel, tin, zinc, nickel, magnesium, manganese, titanium, beryllium or any of the alloys thereof, ceramics or ceramic matrix composites with high thermal conductivity, carbon or graphite or any type of plastic.

Optionally, in addition, it can incorporate one or more sheets made of insulating materials, preferably polyurethane foam, on the base of the inner faces of the body (133) and of the lid (139), with a thickness of 10 to 40 mm, of preferably 30 mm and on the walls of both with a thickness of 10 to 40 mm, preferably 20 mm and a density of 20 kg/m2 to 50 kg/m2, preferably 30 kg/m2, in both parts.

Optionally, it comprises one or more thermal covers (31) forming part of the body of the thermal mop, as well as the heat shield (10) and/or some other type of insulating sheet (134).

The mop comprises a telescopic or non-telescopic pole (135) that is fastened to the mop body by means of a frame or a threaded piece, preferably by means of a threaded piece (136).

In any case, the body (133) of the mop contains fastening systems of the autonomous thermal device, such as an independent frame with tabs or grate that is fixed to the base of the container, preferably tabs (137) on the lower edge of the body.

The body (133) is adjusted to the lid (138) by means of a pressure closure system, velcro, snaps, lever with or without a padlock or any other, preferably by pressure and can contain an automated motorised robot with wheels.

Optionally, it can also have a temperature indicator, preferably with an acoustic alarm.

Logically, on the body (133) of the mop there may be fastened a disposable or non-disposable mop made of materials selected from the group consisting of microfibre, polyester, cotton, non-woven fabric, preferably silver or copper metallised fabric, or any other material used for the manufacture of disposable or non-disposable mops, as well as having the shapes and sizes of both the body and the lid to adapt to any type of domestic or professional mop, serving for the cleaning and thermal sanitisation of any surface in order to eliminate mites, microorganisms, insects, ectoparasites and fungi, or to vaporise molecules, substances or compounds on said surfaces.

## Claims

1. A disposable autonomous thermal device **characterised in that** it comprises:
- a body in the form of a container (1) comprising one or more sheets (5);
- an exothermal composition (4), incorporated into the body of the container (1), wherein the exothermal composition (4) comprises:
- from 1% to 90% by weight of one or more inorganic or organic acids, an acid of any acidic salt or a mixture of the same;
- from 1% to 90% by weight of one or more inorganic bases selected from one or more hydroxides or a base of any basic salt or a mixture of the same;
- from 1% to 90% by weight of one or more moisture-absorbing materials;
- and an aqueous liquid (6) separated from the exothermal composition (4), inside or outside the container (1),
wherein, when the exothermal composition (4) of the container (1) is combined with the aqueous liquid (6), manually or by means of an auxiliary activating element (50), an exothermal neutralising reaction is produced, and wherein the exothermal composition (4) contains between 20% and 50% by weight of the acidic ingredient; between 35% and 60% by weight of the basic ingredient; between 1% and 40% by weight of moisture-absorbing materials; **in that** the acidic ingredient is a citric acid; **in that** the basic ingredient is a calcium hydroxide; and **in that** the moisture-absorbing materials are selected from any from among particulate wood, pelletised wood, sodium polyacrylate, cellulose, starches, corn starch, potato starch, katakuri starch, collagen, agar, carrageenan, gelatin, sodium pyrophosphate, furcelleran, polysaccharides, pectin, proteins, amylum, sago, locust bean gum, xanthan gum, guar gum, vegetable gums, arrowroot, tapioca, alginine and mixtures thereof.

2. The disposable autonomous thermal device according to claim 1, wherein the exothermal composition (4) comprises between 10% and 30% by weight of particulate wood; in that the particulate wood can be from silvicultural, agricultural, agro-industrial, energy or forestry species selected from the group consisting of olive pits, nut shells, fir, birch, acacia, aspen, carob, cork oak, larch, almond tree, bamboo, cedar, cypress, poplar, juniper, holm oak, eucalyptus, flamboyant, ash, fruit trees, beech, pine, walnut, elm, olive, oak, teak, among others, and mixtures thereof; and in that the particulate wood comprises from 1% to 20% by weight of moisture.

3. The disposable autonomous thermal device according to any of claims 1 to 2, wherein the exothermal composition (4) has a solid consistency, with the possibility of being in the form of powder, granules, pellets or tablets; and in that the ingredients of the exothermal composition (4): are either unmixed and separated from each other inside the container (1) of the device, some being loose inside said container (1) and the others inside another container or inner bag of water-soluble material that, in turn, is inside the main container (1) of the device; or they are unmixed and separated from each other inside the container (1) of the device, some being inside a first container or inner bag of water-soluble material and the others inside a second container or other inner bag of water-soluble material; or they are mixed and bonded inside the container (1) of the device in an inner bag of water-soluble material incorporated in turn inside said container (1) of the device.

4. The disposable autonomous thermal device according to any of claims 1 to 3, wherein the aqueous liquid (6) comprised by the container (1) of the device is selected from the group consisting of water, distilled water, double distilled water, deionised water, mineral water, spring water, water with salts, brackish water, salt water, or any of the mixtures thereof, preferably distilled water; in that the aqueous liquid (6) is added to the exothermal composition (4) from 20 to 30 parts by weight of this composition in order to generate the heat by a neutralising reaction and it is a solution with the acid, a solution with one or more bases or a solution with hydrogen peroxide or alcohol.

5. The disposable autonomous thermal device according to claim 4, wherein the aqueous liquid (6) is a solution with hydrogen peroxide, wherein the hydrogen peroxide of said solution comprises from 0.20% by weight to 10% of the solution; contains a concentration of 1.5% (5 volumes) to a concentration of 50% (200 volumes); in that said aqueous liquid (6) is contained in a secondary container (7) to the main container (1) of the device; in that the secondary container (7) is inside the main container (1); in that said container is selected from the group consisting of a vial, bottle, canister, ampoule, glass, bag, terrine and syringe, being in any case preferably a breakable bag or a breakable receptacle and comprises one or more sheets of polymeric material sealed forming a flexible bag with a thickness between 20 and 150 microns.

6. The disposable autonomous thermal device according to any of claims 1 to 5, wherein the main container (1) of the device comprises one or more sheets (5) of polymeric material sealed forming a flexible bag adaptable to the shape of any part of the human body, animal or object to be heated and, either it is of a water-soluble composition selected from the group consisting of Polyvinyl Alcohol (PVA/PVAL/POVH) and derivatives, or it is not water-soluble.

7. The disposable autonomous thermal device according to claim 6, wherein one or more sheets (5) of the main container (1) of the device is Polyester (PET) - Aluminium (AL) - Polyethylene (PE) and, the thickness of said polymeric material and the thickness is between 20 and 150 microns; in that one or more sheets (5) of the main container (1) of the device are sealed inside same enabling subdivisions; in that the container (1) comprises an envelope or wrapper, a vacuum bag or one or more sheets of air bubbles; in that the container (1) comprises a closure system on one or more sides thereof; in that one or more sheets (5) of polymeric material of the container (1) of the device or main container are hermetically sealed forming a closed bag with a high degree of vacuum; and in that container (1) has adhered to the outside, or in the form of a cover, support or wrapping that comprises, or which can be incorporated therein, pharmaceutical compounds, insecticides, essential oils and non-active and active aromatic compounds, extracts and natural plant compounds such as tea oils, neem oil, essential lemon eucalyptus oil, citronella oil, savory, garlic, basil, bergamot, cinnamon, cardamom, onion, cedar, coriander, citronella, clove, juniper, tarragon, eucalyptus, geranium, lemongrass, fennel, bay leaf, lavender, lemon, marjoram, lemon balm, mint, mustard, orange, oregano, rosewood, paprika, parsley, pine, grapefruit, rosemary, sage, soy, thyme, vanilla, etc. or mixtures thereof in order to increase the effectiveness of therapeutic uses, thermal sanitisation, to flavour, insecticide or insect repellent.

8. The disposable autonomous thermal device according to any of claims 1 to 7, wherein the container (1) has means of fastening to parts of the body or objects, such as velcro, adhesive, straps, tapes and belts, among others; in that the container (1) is joined to the container (1) of other identical or similar autonomous thermal devices, forming a single body of several of the same; in that it further comprises a heat shield (10) that insulates from the cold and distributes the heat generated by the container (1) of said device, which comprises one or more layers of bonded superimposed materials and fabrics; in that one or more materials of the layers of said heat shield (10) are selected from the group consisting of: wool, polyester, metallic materials such as silver or copper, aerogel, neoprene, wool wadding, acetate, polyester or cotton, among others and any of the mixtures thereof, preferably three layers of neoprene, aerogel and wool; in that in that one or more metallic materials of any of the layers of the heat shield (10) are silver or copper, in percentages from 5 to 100%, on one or the two faces; in that one or more superimposed layers of the heat shield (10) are joined by any joining system, selected from the group consisting of: stitching, buttons, clasps, zip, velcro, laces and eyelets, studs, glue, hot-melt spray, glue, heat sealing, among others, depending on the object or garment to which it is adhered; in that said heat shield (10) is incorporated and coupled to any non-textile or textile object, item of clothing for people or animals already marketed, located on them in the areas near the thermal device, covering or located on the container (1); and in that the heat shield (10) is manufactured in the shape of objects or items of clothing.

9. The disposable autonomous thermal device according to any of claims 1 to 8, further comprising a thermal cover (31) that distributes the heat generated by the thermal device to any object or parts thereof, surfaces, living beings, food or beverages, which comprises one or more insulating sheets (34) between which a hollow (33) is defined that houses the container (1) of the device and a closure system; in that said one or more sheets (34) are made of one or more types of materials selected from the group comprising polymers of inorganic, natural organic and synthetic organic origin; in that one or more sheets (34) are made of materials selected from the group consisting of polyurethane or polyester or PVC or polyethylene foam; in foams or agglomerate or extruded or in memory foam; elastomers such as neoprene, lycra or spandex; cork, cardboard, rubber, aerogel, or they consist of a fabric or mesh (35) that incorporates metallic materials such as silver or copper, in percentages of 5 to 100%, preferably in percentages of these metals between 5 and 40%, or mixtures thereof; in that the cover (31) has polyurethane foam sheets (34), which are separated leaving a hollow (33) that houses the container (1) of the device; being arranged such that there is a sheet of foam rubber covered by fabric on both faces, a hollow (33) that houses the container (1) of the autonomous thermal device and a sheet of aerogel covered by fabric on both faces; in that said polyurethane foam sheets (34) have a thickness of between 1 mm and 300mm and have a density between 5 kg/m3 and 70 kg/m3, preferably between 10 kg/m3 and 30 kg/m3; and in that one or both polyurethane foam sheets (34) are of the very flexible soft type.

10. The disposable autonomous thermal device according to claim 9, wherein the cover (31) is covered inside, outside or both by a fabric or a mesh (35) wherein one or more materials of said fabric or mesh (35) are selected from the group consisting of any natural textile material such as cotton, wool, silk, leather, micro-wool and linen among others, or synthetics such as polyamide, nylon, polyester, PVC, trilaminate, tetralaminate, vinyl, neoprene, PU, lycra or spandex, copper or silver metallic fabrics, silicone, latex, neoprene, anti-mite fabric, polyester, aerogel, non-woven fabric, aluminised plastic or not, nylon, vinyl among others or any of the mixtures thereof; in that the cover (31) contains, on said fabric or mesh (35), an additional neoprene coating; in that the fabric or mesh (35) contains metallic materials such as silver or copper, in percentages of 5 and 40%; in that the cover (31) comprises a zip (32), velcro (36), a flap with velcro (37), magnets, clasps, snaps or others to insulate inside the thermal device; in that it comprises fastening systems to fasten or adapt to the body of people, animals, items of clothing, bedding, sleeping bags, harnesses and any object to be heated, selected from the group consisting of straps, tapes, belts, velcro, glue, adhesive, or a magnetic sheet (39) or magnets (40), or with buckles, sewing etc.; and in that the cover (31) has a configuration that comprises one or more tabs in the upper (41), lower (42) and lateral (43) position, with closure systems, that serve to adhere and adjust to objects such as straps (219) of a harness.

11. The disposable autonomous thermal device according to any of claims 9 or 10, wherein the cover (31) comprises an anti- radiation pocket (38), to prevent radiation from mobile devices and theft of data of same or of bank cards, etc.; in that the cover (31) contains a heat shield (10) as described in claims 39 to 44; in that the cover (31) contains a temperature indicator (44); in that the cover (31) contains one or more photovoltaic electrical generators (45) to generate and supply electrical energy to charge any battery or directly any electronic mobile device such as mobile phones, computers or tablets among others, or any electrical or electronic apparatus; in that the cover (31) contains one or more thermoelectric generators (46) that use the heat generated by the thermal device, the container (1) of which is incorporated in said cover (31) to generate and supply electrical energy to charge any battery or directly any electronic mobile device such as mobile phones, computers or tablets among others, or any electrical or electronic apparatus; and in that the cover (31) contains one or more terminals for charging electronic devices and/or one or more batteries.

12. The disposable autonomous thermal device according to any of claims 9 to 11, wherein the cover (31) contains a wrapping that comprises, or which can be incorporated therein, pharmaceutical compounds, insecticides, essential oils and non-active and active aromatic compounds, extracts and natural plant compounds such as tea oils, neem oil, essential lemon eucalyptus oil, citronella oil, savory, garlic, basil, bergamot, cinnamon, cardamom, onion, cedar, coriander, citronella, clove, juniper, tarragon, eucalyptus, geranium, lemongrass, fennel, bay leaf, lavender, lemon, marjoram, lemon balm, mint, mustard, orange, oregano, rosewood, paprika, parsley, pine, grapefruit, rosemary, sage, soy, thyme, vanilla, etc. or mixtures thereof; in that it comprises an activator (50) applicable to the cover (31) which comprises a lid (47) with one or more strikers (48) and a base (49), both joined on the outside of the opposite faces of the thermal cover (31), in such a way that, when the lid (47) is pressed, the striker or strikers (48) pass through the thermal cover (31), the sheet (5) of the container (1) of the thermal device and the secondary container (7), and the aqueous liquid (6) comes out contacting the reagents of the exothermal composition (4) activating the exothermal reaction; in that the cover (31) is manufactured in the shape of any part of the body of people or animals in order to be directly coupled to same; and in that the cover (31) is manufactured in the shape of objects or items of clothing or to be incorporated and coupled to any non-textile or textile object, item of clothing for people or animals.

13. The disposable autonomous thermal device according to any of claims 9 to 12, wherein the container (1) is incorporated, housed in the cover (31) including or not the heat shield (10), to the straps (219) of a harness to attach it to the body of a user; and in that the two straps (219) of the harness and/or one or more thermal covers (31) incorporated into said harness contain accessories such as material-holder rings (223), padded protectors (224), pockets, carabiners and pins.

14. An object comprising a disposable autonomous thermal device according to any of the preceding claims.

15. The object according to claim 14, consisting of a thermal mop that comprises a body (133) provided with a hollow to house the container (1) of the device with a closing lid (138); in that the material of the body and cover of said thermal mop is selected from the group consisting of gold, silver, platinum, copper, aluminium, iron, lead, steel, tin, zinc, nickel, magnesium, manganese, titanium, beryllium or any of the alloys thereof, ceramics or ceramic matrix composites with high thermal conductivity, carbon or graphite or any type of plastic; in that, in addition, it incorporates one or more sheets made of insulating materials, such as polyurethane foam, on the base of the inner faces of the body (133) and of the lid (139); in that it comprises one or more thermal covers (31) forming part of the body of the thermal mop; in that it comprises a heat shield (10) forming part of the body of the thermal mop; in that the body (133) of the mop contains fastening systems of the container (1) of the autonomous thermal device, such as an independent frame with tabs (137) on the lower edge of the body; in that the body (133) is adjusted to the lid (138) by means of a pressure closure system, velcro, snaps, lever with or without a padlock or other; in that the mop contains an automated motorised robot with wheels; and in that the mop contains a temperature indicator with an acoustic alarm.

## Patentansprüche

1. Autonome thermische Einwegvorrichtung, **dadurch gekennzeichnet, dass** sie umfasst:
- einen Körper in Form eines Behälters (1), der eine oder mehrere Lagen (5) umfasst;
- eine exotherme Zusammensetzung (4), die in den Körper des Behälters (1) einbezogen ist, wobei die exotherme Zusammensetzung (4) umfasst:
- von 1 Gew.-% bis 90 Gew.-% einer oder mehrerer anorganischer oder organischer Säuren, einer Säure eines beliebigen sauren Salzes oder eine Mischung derselben;
- von 1 Gew.-% bis 90 Gew.-% einer oder mehrerer anorganischer Basen, die ausgewählt sind aus einem oder mehreren Hydroxiden oder einer Base eines beliebigen basischen Salzes oder einer Mischung derselben;
- von 1 Gew.-% bis 90 Gew.-% eines oder mehrerer feuchtigkeitsabsorbierender Materialien;
- und eine von der exothermen Zusammensetzung (4) getrennte wässrige Flüssigkeit (6) innerhalb oder außerhalb des Behälters (1),
wobei, wenn die exotherme Zusammensetzung (4) des Behälters (1) mit der wässrigen Flüssigkeit (6) kombiniert wird, manuell oder mittels eines aktivierenden Hilfselements (50), eine exotherme Neutralisierungsreaktion erzeugt wird, und wobei die exotherme Zusammensetzung (4) zwischen 20 Gew.-% und 50 Gew.-% des sauren Bestandteils enthält; zwischen 35 Gew.-% und 60 Gew.-% des badischen Bestandteils; zwischen 1 Gew.-% und 40 Gew.-% feuchtigkeitsabsorbierender Materialien; dadurch, dass der saure Bestandteil eine Zitronensäure ist; dadurch, dass der basische Bestandteil ein Calciumhydroxid ist; und dadurch, dass die feuchtigkeitsabsorbierenden Materialien ausgewählt sind aus Holzpartikeln, pelletiertem Holz, Natriumpolyacrylat, Cellulose, Stärken, Maisstärke, Kartoffelstärke, Katakuristärke, Kollagen, Agar, Carrageen, Gelatine, Natriumpyrophosphat, Furcelleran, Polysaccharide, Pektin, Proteinen, Amylum, Sago, Johannisbrotkernmehl, Xanthan, Guarkernmehl, pflanzlichen Gummis, Pfeilwurz, Tapioka, Alginin und Mischungen davon.

2. Autonome thermische Einwegvorrichtung nach Anspruch 1, wobei die exotherme Zusammensetzung (4) zwischen 10 Gew.-% und 30 Gew.-% partikelförmiges Holz umfasst; dadurch, dass das partikelförmige Holz von forstwirtschaftlichen, landwirtschaftlichen, agroindustriellen, energetischen oder forstwirtschaftlichen Arten stammen kann, die aus der Gruppe ausgewählt sind, die unter anderem aus Olivenkernen, Nussschalen, Tanne, Birke, Akazie, Espe, Johannisbrotbaum, Tanne, Birke, Akazie, Espe, Johannisbrotbaum, Lärche, Mandelbaum, Bambus, Zeder, Zypresse, Pappel, Wacholder, Steineiche, Eukalyptus, Esche, Obstbäumen, Buche, Kiefer, Walnuss, Ulme, Olive, Eiche, Teak sowie Mischungen davon besteht; und dadurch, dass das partikelförmige Holz einen Feuchtigkeitsgehalt von 1 Gew.-% bis 20 Gew.-% umfasst.

3. Autonome thermische Einwegvorrichtung nach einem der Ansprüche 1 bis 2, wobei die exotherme Zusammensetzung (4) eine feste Konsistenz mit der Möglichkeit aufweist, in Form von Pulver, Granulat, Pellets oder Tabletten vorzuliegen; und dadurch, dass die Bestandteile der exothermen Zusammensetzung (4): entweder unvermischt und voneinander getrennt innerhalb des Behälters (1) der Vorrichtung sind, wobei einige lose innerhalb des Behälters (1) und die anderen innerhalb eines anderen Behälters oder inneren Beutels aus wasserlöslichem Material vorliegen, der sich wiederum innerhalb des Hauptbehälters (1) der Vorrichtung befindet; oder sie sind im Inneren des Behälters (1) der Vorrichtung unvermischt und voneinander getrennt, wobei einige in einem ersten Behälter oder inneren Beutel aus wasserlöslichem Material und die anderen in einem zweiten Behälter oder anderen inneren Beutel aus wasserlöslichem Material vorliegen; oder sie sind im Inneren des Behälters (1) der Vorrichtung in einem inneren Beutel aus wasserlöslichem Material gemischt und gebunden, der wiederum in den Behälter (1) der Vorrichtung einbezogen ist.

4. Autonome thermische Einwegvorrichtung nach einem der Ansprüche 1 bis 3, wobei die wässrige Flüssigkeit (6), die der Behälter (1) der Vorrichtung enthält, aus der Gruppe ausgewählt ist, die aus Wasser, destilliertem Wasser, zweifach destilliertem Wasser, entionisiertem Wasser, Mineralwasser, Quellwasser, Wasser mit Salzen, Brackwasser, Salzwasser oder einer beliebigen der Mischungen davon besteht, vorzugsweise aus destilliertem Wasser; dadurch, dass die wässrige Flüssigkeit (6) der exothermen Zusammensetzung (4) zu 20 bis 30 Gewichtsteilen dieser Zusammensetzung zugesetzt ist, um die Wärme durch eine neutralisierende Reaktion zu erzeugen, und es sich um eine Lösung mit der Säure, eine Lösung mit einer oder mehreren Basen oder eine Lösung mit Wasserstoffperoxid oder Alkohol handelt.

5. Autonome thermische Einwegvorrichtung nach Anspruch 4, wobei die wässrige Flüssigkeit (6) eine Lösung mit Wasserstoffperoxid ist, wobei das Wasserstoffperoxid der Lösung von 0,20 Gew.-% bis 10 % der Lösung umfasst; eine Konzentration von 1,5 % (5 Volumina) bis zu einer Konzentration von 50 % (200 Volumina) enthält; dadurch, dass die wässrige Flüssigkeit (6) in einem Nebenbehälter (7) zum Hauptbehälter (1) der Vorrichtung enthalten ist; dadurch, dass sich der Nebenbehälter (7) im Inneren des Hauptbehälters (1) befindet; dadurch, dass der Behälter aus der Gruppe ausgewählt ist, die aus einem Fläschchen, einer Flasche, einem Kanister, einer Ampulle, einem Glas, einem Beutel, einer Terrine und einer Spritze besteht, wobei es sich in jedem Fall vorzugsweise um einen zerbrechlichen Beutel oder ein zerbrechliches Gefäß handelt und eine oder mehrere Lagen aus polymerem Material umfasst, die unter Bildung eines flexiblen Beutels mit einer Dicke zwischen 20 und 150 Mikron versiegelt sind.

6. Autonome thermische Einwegvorrichtung nach einem der Ansprüche 1 bis 5, wobei der Hauptbehälter (1) der Vorrichtung eine oder mehrere Lagen (5) aus polymerem Material umfasst, die versiegelt sind und einen flexiblen Beutel bilden, der an die Form eines beliebigen Teils des zu erwärmenden menschlichen Körpers, Tieres oder Gegenstandes anpassbar ist und der entweder aus einer wasserlöslichen Zusammensetzung besteht, die aus der Gruppe ausgewählt ist, die aus Polyvinylalkohol (PVA/PVAL/POVH) und Derivaten besteht, oder nicht wasserlöslich ist.

7. Autonome thermische Einwegvorrichtung nach Anspruch 6, wobei eine oder mehrere Schichten (5) des Hauptbehälters (1) der Vorrichtung aus Polyester (PET) - Aluminium (AL) - Polyethylen (PE) bestehen und die Dicke des Polymermaterials und die Dicke zwischen 20 und 150 Mikrometern liegt; dadurch, dass eine oder mehrere Lagen (5) des Hauptbehälters (1) der Vorrichtung im Inneren derselben Unterteilungen versiegelt sind, die eine Unterteilung ermöglichen; dadurch, dass der Behälter (1) eine Hülle oder Umhüllung, einen Vakuumbeutel oder eine oder mehrere Lagen mit Luftblasen umfasst; dadurch, dass der Behälter (1) an einer oder mehreren Seiten davon ein Verschlusssystem umfasst; dadurch, dass eine oder mehrere Lagen (5) des polymeren Materials des Behälters (1) der Vorrichtung oder des Hauptbehälters hermetisch versiegelt sind und einen geschlossenen Beutel mit einem hohen Vakuumgrad bilden; und dadurch, dass der Behälter (1) an der Außenseite oder in Form einer Hülle, eines Trägers oder einer Umhüllung anhaftet, die bzw. der pharmazeutische Verbindungen, Insektizide, ätherische Öle und nichtaktive und aktive aromatische Verbindungen, Extrakte und natürliche Pflanzenverbindungen wie zum Beispiel Teeöle, Neemöl, ätherisches Zitronen-Eukalyptusöl Citronellaöl, Bohnenkraut, Knoblauch, Basilikum, Bergamotte, Zimt, Kardamom, Zwiebel, Zeder, Koriander, Citronella, Nelke, Wacholder, Estragon, Eukalyptus, Geranie, Zitronengras, Fenchel, Lorbeer, Lavendel, Zitrone, Majoran, Zitronenmelisse, Minze, Senf, Orange, Oregano, Rosenholz, Paprika, Petersilie, Kiefer, Grapefruit, Rosmarin, Salbei, Soja, Thymian, Vanille, usw. oder Mischungen davon umfasst, um die Wirksamkeit therapeutischer Anwendungen, der thermischen Desinfektion, des Aromas, des Insektizids oder der Insektenabwehr zu erhöhen.

8. Autonome thermische Einwegvorrichtung nach einem der Ansprüche 1 bis 7, wobei der Behälter (1) Mittel zum Befestigen an Körperteilen oder Gegenständen aufweist wie unter anderem zum Beispiel Klettverschluss, Klebstoff, Riemen, Bänder und Gürtel aufweist; dadurch, dass der Behälter (1) mit den Behältern (1) anderer identischer oder ähnlicher autonomer thermischer Geräte verbunden ist und einen einzigen Körper aus mehreren derselben bildet; dadurch, dass sie ferner einen Hitzeschild (10) umfasst, der gegen Kälte isoliert und die durch den Behälter (1) der Vorrichtung erzeugte Wärme verteilt, der eine oder mehrere Schichten aus miteinander verbundenen übereinanderliegenden Materialien und Geweben umfasst; dadurch, dass ein oder mehrere Materialien der Schichten des Hitzeschildes (10) ausgewählt sind aus der Gruppe, die aus Wolle, Polyester, metallischen Materialien wie unter anderem zum Beispiel Silber oder Kupfer, Aerogel, Neopren, Wollwatte, Acetat, Polyester oder Baumwolle und beliebigen Mischungen davon besteht, vorzugsweise aus drei Schichten aus Neopren, Aerogel und Wolle; dadurch, dass dadurch, dass ein oder mehrere metallische Werkstoffe einer der Schichten des Hitzeschildes (10) auf einer oder den beiden Seiten Silber oder Kupfer in Anteilen von 5 bis 100 % sind; dadurch, dass eine oder mehrere übereinanderliegende Schichten des Hitzeschildes (10) durch ein beliebiges Verbindungssystem verbunden sind, das aus der Gruppe ausgewählt wird, die je nach dem Gegenstand oder Kleidungsstück, an dem es anhaftet, unter anderem besteht aus: Nähten, Knöpfen, Schnallen, Reißverschlüsse, Klettverschlüssen, Schnürsenkeln und Ösen, Nieten, Klebstoff, Heißklebespray, Leim, Heißversiegelung; dadurch, dass der Hitzeschild (10) in einen beliebigen nichttextilen oder textilen Gegenstand, ein Kleidungsstück für Menschen oder Tiere, der/das bereits vermarktet wird, einbezogen und mit diesem gekoppelt ist, der/das sich in der Nähe der thermischen Vorrichtung befindet und den Behälter (1) bedeckt oder sich an diesem befindet; und dadurch, dass der Hitzeschild (10) in Form von Gegenständen oder Kleidungsstücken hergestellt ist.

9. Autonome thermische Einwegvorrichtung nach einem der Ansprüche 1 bis 8, die ferner eine thermische Abdeckung (31) umfasst, die die durch die thermische Vorrichtung erzeugte Wärme auf beliebige Gegenstände oder Teile davon, Oberflächen, Lebewesen, Lebensmittel oder Getränke verteilt, die eine oder mehrere isolierende Lagen (34) umfasst, zwischen denen ein Hohlraum (33) definiert ist, der den Behälter (1) der Vorrichtung und ein Verschlusssystem aufnimmt; dadurch, dass die eine oder mehreren Lagen (34) aus einer oder mehreren Arten von Materialien bestehen, die aus der Gruppe ausgewählt sind, die Polymere anorganischen, natürlichen organischen und synthetischen organischen Ursprungs umfasst; dadurch, dass eine oder mehrere Lagen (34) aus Materialien bestehen, die aus der Gruppe ausgewählt sind, die aus Polyurethan- oder Polyester- oder PVC-Schaum oder Polyethylenschaum; aus Schaumstoffen oder Agglomerat oder extrudiert oder aus Memory-Schaum; Elastomeren wie Neopren, Lycra oder Spandex; Kork, Pappe, Gummi, Aerogel besteht, oder sie aus einem Gewebe oder Netz (35) bestehen, das metallische Materialien wie zum Beispiel Silber oder Kupfer in Anteilen von 5 bis 100 %, vorzugsweise in Anteilen dieser Metalle zwischen 5 und 40 %, oder Mischungen davon enthält; dadurch, dass die Abdeckung (31) aus Polyurethanschaumlagen (34) besteht, die voneinander getrennt sind, sodass ein Hohlraum (33) entsteht, der den Behälter (1) der Vorrichtung aufnimmt; die so angeordnet ist, dass eine beidseitig mit Stoff bedeckte Schaumgummilage, ein Hohlraum (33), der den Behälter (1) der autonomen Wärmevorrichtung aufnimmt, und eine Aerogel-Lage vorliegen, die auf beiden Seiten mit Stoff bedeckt ist; dadurch, dass die Polyurethanschaumlagen (34) eine Dicke zwischen 1 mm und 300 mm und eine Dichte zwischen 5 kg/m3 und 70 kg/m3, vorzugsweise zwischen 10 kg/m3 und 30 kg/m3 aufweisen; und dadurch, dass eine oder beide Polyurethanschaumlagen (34) vom sehr flexiblen, weichen Typ sind.

10. Autonome thermische Einwegvorrichtung nach Anspruch 9, wobei die Abdeckung (31) innen, außen oder beides durch ein Gewebe oder ein Netz (35) bedeckt ist, wobei ein oder mehrere Materialien des Gewebes oder Netzes (35) aus der Gruppe ausgewählt sind, die aus einem natürlichen Textilmaterial wie unter anderem zum Beispiel Baumwolle, Wolle, Seide, Leder, Mikrowolle und Leinen oder synthetischen Materialien wie unter anderem zum Beispiel Polyamid, Nylon, Polyester, PVC, Trilaminat, Tetralaminat, Vinyl, Neopren, PU, Lycra oder Spandex, Kupfer- oder Silbermetallgewebe, Silikon, Latex, Neopren, Anti-Milben-Gewebe, Polyester, Aerogel, Vliesstoff, aluminisiertem oder nichtaluminisiertem Kunststoff, Nylon, Vinyl und anderen oder beliebigen Mischungen davon besteht; dadurch, dass die Abdeckung (31) auf dem Gewebe oder Netz (35) eine zusätzliche Neoprenbeschichtung enthält; dadurch, dass das Gewebe oder Netz (35) metallische Materialien wie zum Beispiel Silber oder Kupfer in Anteilen von 5 und 40 % enthält; dadurch, dass die Abdeckung (31) einen Reißverschluss (32), einen Klettverschluss (36), eine Klappe mit Klettverschluss (37), Magneten, Schnallen, Druckknöpfen oder anderem umfasst, um das Innere der thermischen Vorrichtung zu isolieren; dadurch, dass sie Befestigungssysteme zum Befestigen oder Anpassen an den Körper von Menschen, Tieren, an Kleidungsstücke, Bettzeug, Schlafsäcke, Geschirre und beliebigen zu beheizenden Gegenständen umfasst, die aus der Gruppe ausgewählt sind, die aus Riemen, Bändern, Gürteln, Klettverschluss, Leim, Klebstoff oder einer magnetischen Lage (39) oder Magneten (40) oder mit Schnallen, Nähten usw. besteht; und dadurch, dass die Abdeckung (31) eine Konfiguration aufweist, die eine oder mehrere Laschen in der oberen (41), unteren (42) und seitlichen (43) Position mit Verschlusssystemen umfasst, die dazu dienen, an Gegenständen wie zum Beispiel Riemen (219) eines Gurtzeugs zu haften und sich anzupassen.

11. Autonome thermische Einwegvorrichtung nach einem der Ansprüche 9 oder 10, wobei die Abdeckung (31) eine Strahlungsschutztasche (38) umfasst, um Strahlung aus mobilen Vorrichtungen und den Diebstahl von Daten derselben oder von Bankkarten usw. zu verhindern; dadurch, dass die Abdeckung (31) einen Hitzeschild (10) nach den Ansprüchen 39 bis 44 enthält; dadurch, dass die Abdeckung (31) eine Temperaturanzeige (44) enthält; dadurch, dass die Abdeckung (31) einen oder mehrere photovoltaische elektrische Generatoren (45) enthält, um elektrische Energie zu erzeugen und zuzuführen, um eine beliebige Batterie oder direkt eine elektronische mobile Vorrichtung wie unter anderem zum Beispiel Mobiltelefone, Computer oder Tablets oder ein beliebiges elektrisches oder elektronisches Gerät zu laden; dass die Abdeckung (31) einen oder mehrere thermoelektrische Generatoren (46) enthält, die die durch die thermische Vorrichtung erzeugte Wärme nutzen, deren Behälter (1) in die Abdeckung (31) einbezogen ist, um eine beliebige Batterie oder direkt eine elektronische mobile Vorrichtung wie unter anderem zum Beispiel Mobiltelefone, Computer oder Tablets oder ein beliebiges elektrisches oder elektronisches Gerät zu laden; und dadurch, dass die Abdeckung (31) einen oder mehrere Anschlüsse zum Laden elektronischer Vorrichtungen und/oder einer oder mehrerer Batterien enthält.

12. Autonome thermische Vorrichtung nach einem der Ansprüche 9 bis 11, wobei die Abdeckung (31) eine Umhüllung enthält, die pharmazeutische Verbindungen, Insektizide, ätherische Öle und nichtaktive und aktive aromatische Verbindungen, Extrakte und natürliche Pflanzenverbindungen wie zum Beispiel Teeöle, Neemöl, ätherisches Zitronen-Eukalyptusöl Citronellaöl, Bohnenkraut, Knoblauch, Basilikum, Bergamotte, Zimt, Kardamom, Zwiebel, Zeder, Koriander, Citronella, Nelke, Wacholder, Estragon, Eukalyptus, Geranie, Zitronengras, Fenchel, Lorbeer, Lavendel, Zitrone, Majoran, Zitronenmelisse, Minze, Senf, Orange, Oregano, Rosenholz, Paprika, Petersilie, Kiefer, Grapefruit, Rosmarin, Salbei, Soja, Thymian, Vanille, usw. oder Mischungen davon umfasst oder darin einbezogen sein kann; dadurch, dass sie einen Aktivator (50) umfasst, der auf die Abdeckung (31) anwendbar ist, die einen Deckel (47) mit einem oder mehreren Schlagbolzen (48) und ein Unterteil (49) umfasst, die beide auf der Außenseite der gegenüberliegenden Seiten der thermischen Abdeckung (31) verbunden sind, sodass, wenn der Deckel (47) gedrückt wird, der Schlagbolzen oder die Schlagbolzen (48) die thermische Abdeckung (31), die Lage (5) des Behälters (1) der thermischen Vorrichtung und den Nebenbehälter (7) durchdringen und die wässrige Flüssigkeit (6) austritt und mit den Reagenzien der exothermen Zusammensetzung (4) in Kontakt gelangt, wodurch die exotherme Reaktion aktiviert wird; dadurch, dass die Abdeckung (31) in Form eines beliebigen Körperteils von Menschen oder Tieren hergestellt ist, um direkt mit diesem gekoppelt zu werden; und dadurch, dass die Abdeckung (31) in Form von Gegenständen oder Kleidungsstücken hergestellt ist oder in einen beliebigen nichttextilen oder textilen Gegenstand, ein Kleidungsstück für Menschen oder Tiere einbezogen und mit diesem gekoppelt werden soll.

13. Autonome thermische Einwegvorrichtung nach einem der Ansprüche 9 bis 12, wobei der Behälter (1), der einschließlich des Hitzeschildes (10) oder ohne dieses in der Abdeckung (31) untergebracht ist, in die Riemen (219) eines Gurtzeugs einbezogen ist, um ihn am Körper eines Benutzers anzubringen; und dadurch, dass die beiden Riemen (219) des Gurtzeugs und/oder eine oder mehrere in das Gurtzeug einbezogene thermische Abdeckungen (31) Zubehörteile wie zum Beispiel Materialhalteringe (223), gepolsterte Protektoren (224), Taschen, Karabiner und Stifte enthalten.

14. Gegenstand, der eine autonome thermische Einwegvorrichtung nach einem der vorhergehenden Ansprüche umfasst.

15. Gegenstand nach Anspruch 14, der aus einem thermischen Mopp besteht, der einen Körper (133) umfasst, der mit einem Hohlraum zum Aufnehmen des Behälters (1) der Vorrichtung mit einem Verschlussdeckel (138) versehen ist; dadurch, dass das Material des Körpers und der Abdeckung des thermischen Mopps aus der Gruppe ausgewählt ist, die aus Gold, Silber, Platin, Kupfer, Aluminium, Eisen, Blei, Stahl, Zinn, Zink, Nickel, Magnesium, Mangan, Titan, Beryllium oder einer Legierung davon, Keramik oder Verbundwerkstoffen mit keramischer Matrix und hoher Wärmeleitfähigkeit, Kohlenstoff oder Graphit oder einer beliebigen Art von Kunststoff besteht; dadurch, dass er außerdem eine oder mehrere Lagen aus isolierendem Material wie zum Beispiel Polyurethanschaum, am Unterteil der Innenflächen des Körpers (133) und des Deckels (139) aufweist; dadurch, dass er eine oder mehrere thermische Abdeckungen (31) umfasst, die einen Teil des Körpers des thermischen Mops bilden; dadurch, dass er einen Hitzeschild (10) umfasst, der einen Teil des Körpers des thermischen Mopps bildet; dadurch, dass der Körper (133) des Mopps Befestigungssysteme des Behälters (1) der autonomen thermischen Vorrichtung wie zum Beispiel einen unabhängigen Rahmen mit Laschen (137) am unteren Rand des Körpers enthält; dadurch, dass der Körper (133) mittels eines Druckverschlusssystems, eines Klettverschlusses, Druckknöpfen, eines Hebel mit oder ohne Vorhängeschloss oder anderweitig an den Deckel (138) angepasst wird; dadurch, dass der Mopp einen automatischen, motorisierten Roboter mit Rädern enthält; und dadurch, dass der Mopp eine Temperaturanzeige mit einem akustischen Alarm enthält.

## Revendications

1. Dispositif thermique autonome jetable **caractérisé en ce qu'**il comprend :
- un corps sous la forme d'un récipient (1) comprenant une ou plusieurs feuilles (5) ;
- une composition exothermique (4), incorporée dans le corps du récipient (1), dans lequel la composition exothermique (4) comprend :
- de 1 % à 90 % en poids d'un ou plusieurs acides inorganiques ou organiques, d'un acide ou d'un quelconque sel acide ou d'un mélange de ceux-ci ;
- de 1 % à 90 % en poids d'une ou plusieurs bases inorganiques choisies parmi un ou plusieurs hydroxydes ou une base d'un quelconque sel basique ou un mélange de ceux-ci ;
- de 1 % à 90 % en poids d'un ou plusieurs matériaux absorbant l'humidité ;
- et un liquide aqueux (6) séparé de la composition exothermique (4), à l'intérieur ou à l'extérieur du récipient (1),
dans lequel, lorsque la composition exothermique (4) du récipient (1) est combinée avec le liquide aqueux (6), manuellement ou au moyen d'un élément d'activation auxiliaire (50), une réaction de neutralisation exothermique est produite, et dans lequel la composition exothermique (4) contient entre 20 % et 50 % en poids de l'ingrédient acide ; entre 35 % et 60 % en poids de l'ingrédient basique ; entre 1 % et 40 % en poids de matériaux absorbant l'humidité ; **en ce que** l'ingrédient acide est un acide citrique ; **en ce que** l'ingrédient basique est un hydroxyde de calcium ; et **en ce que** les matériaux absorbant l'humidité sont choisis parmi l'un quelconque parmi les particules de bois, le bois granulé, le polyacrylate de sodium, la cellulose, les amidons, l'amidon de maïs, la fécule de pomme de terre, l'amidon de katakuri, le collagène, l'agar, la carraghénane, la gélatine, le pyrophosphate de sodium, le furcellerane, les polysaccharides, la pectine, les protéines, l'amylum, le sagou, la gomme de caroube, la gomme xanthane, la gomme de guar, les gommes végétales, l'arrowroot, le tapioca, l'alginine et des mélanges de ceux-ci.

2. Dispositif thermique autonome jetable selon la revendication 1, dans lequel la composition exothermique (4) comprend entre 10 % et 30 % en poids de particules de bois ; en ce que les particules de bois peuvent être d'espèces sylvicoles, agricoles, agro-industrielles, énergétiques ou forestières choisies dans le groupe constitué par les noyaux d'olive, les coquilles de noix, le sapin, le bouleau, l'acacia, le tremble, la caroube, le chêne-liège, le mélèze, l'amandier, le bambou, le cèdre, le cyprès, le peuplier, le genévrier, le chêne vert, l'eucalyptus, le flamboyant, les cendres, les arbres fruitiers, le hêtre, le pin, la noix, l'orme, l'olive, le chêne, le teck, entre autres, et des mélanges de ceux-ci ; et en ce que les particules de bois comprennent de 1 % à 20 % en poids d'humidité.

3. Dispositif thermique autonome jetable selon l'une quelconque des revendications 1 à 2, dans lequel la composition exothermique (4) a une consistance solide, avec la possibilité d'être sous la forme de poudre, granules, pastilles ou comprimés ; et en ce que les ingrédients de la composition exothermique (4) : sont soit non mélangés et séparés les uns des autres à l'intérieur du récipient (1) du dispositif, certains étant en vrac à l'intérieur dudit récipient (1) et les autres à l'intérieur d'un autre récipient ou sac interne de matériau soluble dans l'eau qui, à son tour, se trouve à l'intérieur du récipient (1) principal du dispositif ; soit ils sont non mélangés et séparés les uns des autres à l'intérieur du récipient (1) du dispositif, certains étant à l'intérieur d'un premier récipient ou sac interne de matériau soluble dans l'eau et les autres à l'intérieur d'un second récipient ou autre sac interne de matériau soluble dans l'eau ; soit ils sont mélangés et liés à l'intérieur du récipient (1) du dispositif dans un sac interne de matériau soluble dans l'eau incorporé à son tour à l'intérieur dudit récipient (1) du dispositif.

4. Dispositif thermique autonome jetable selon l'une quelconque des revendications 1 à 3, dans lequel le liquide aqueux (6) compris par le récipient (1) du dispositif est choisi dans le groupe constitué par l'eau, l'eau distillée, l'eau doublement distillée, l'eau déminéralisée, l'eau minérale, l'eau de source, l'eau avec des sels, l'eau saumâtre, l'eau salée ou un quelconque des mélanges de ceux-ci, de préférence l'eau distillée ; en ce que le liquide aqueux (6) est ajouté à la composition exothermique (4) à raison de 20 à 30 parties en poids de cette composition afin de produire de la chaleur par une réaction de neutralisation et **il** s'agit d'une solution avec l'acide, d'une solution avec une ou plusieurs bases ou d'une solution avec du peroxyde d'hydrogène ou de l'alcool.

5. Dispositif thermique autonome jetable selon la revendication 4, dans lequel le liquide aqueux (6) est une solution de peroxyde d'hydrogène, dans lequel le peroxyde d'hydrogène de ladite solution comprend de 0,20 % en poids à 10 % de la solution ; contient une concentration de 1,5 % (5 volumes) à une concentration de 50 % (200 volumes) ; en ce que ledit liquide aqueux (6) est contenu dans un récipient secondaire (7) au récipient principal (1) du dispositif ; en ce que le récipient secondaire (7) est à l'intérieur du récipient principal (1) ; en ce que ledit récipient est choisi dans le groupe constitué d'un flacon, d'une bouteille, d'un bidon, d'une ampoule, d'un verre, d'un sac, d'une terrine et d'une seringue, étant en tout cas de préférence un sac cassable ou un réceptacle cassable et comprend une ou plusieurs feuilles de matériau polymère scellées formant un sac flexible d'une épaisseur entre 20 et 150 microns.

6. Dispositif thermique autonome jetable selon l'une quelconque des revendications 1 à 5, dans lequel le récipient principal (1) du dispositif comprend une ou plusieurs feuilles (5) de matériau polymère scellées formant un sac flexible pouvant s'adapter à la forme d'une quelconque partie du corps humain, d'un animal ou d'un objet à chauffer et, soit il s'agit d'une composition soluble dans l'eau choisie dans le groupe constitué par l'alcool polyvinylique (PVA/PVAL/POVH) et ses dérivés, soit il n'est pas soluble dans l'eau.

7. Dispositif thermique autonome jetable selon la revendication 6, dans lequel une ou plusieurs feuilles (5) du récipient principal (1) du dispositif sont en polyester (PET) - aluminium (AL) - polyéthylène (PE) et, l'épaisseur dudit matériau polymère et l'épaisseur est entre 20 et 150 microns ; en ce qu'une ou plusieurs feuilles (5) du récipient principal (1) du dispositif sont scellées à l'intérieur des mêmes subdivisions habilitantes ; en ce que le récipient (1) comprend une enveloppe ou un emballage, un sac sous vide ou une ou plusieurs feuilles de bulles d'air ; en ce que le récipient (1) comprend un système de fermeture sur un ou plusieurs côtés de celui-ci ; en ce qu'une ou plusieurs feuilles (5) de matériau polymère du récipient (1) du dispositif ou du récipient principal sont hermétiquement scellées, formant un sac fermé avec un degré élevé de vide ; et en ce que le récipient (1) a adhéré à l'extérieur, ou sous la forme d'un couvercle, un support ou un emballage qui comprend, ou qui peut être incorporé à l'intérieur, des composés pharmaceutiques, des insecticides, des huiles essentielles et des composés aromatiques non actifs et actifs, des extraits et des composés de plantes naturels tels que les huiles de thé, l'huile de neem, l'huile essentielle d'eucalyptus citronné, l'huile de citronnelle, la sarriette, l'ail, le basilic, la bergamote, la cannelle, la cardamome, l'oignon, le cèdre, la coriandre, la citronnelle, le clou de girofle, le genévrier, l'estragon, l'eucalyptus, le géranium, le lemongrass, le fenouil, la feuille de laurier, la lavande, le citron, la marjolaine, la mélisse, la menthe, la moutarde, l'orange, l'origan, le bois de rose, le paprika, le persil, le pin, le pamplemousse, le romarin, la sauge, le soja, le thym, la vanille, etc. ou des mélanges de ceux-ci afin d'augmenter l'efficacité des utilisations thérapeutiques, de l'assainissement thermique, d'aromatiser, d'insecticide ou de répulsif à insectes.

8. Dispositif thermique autonome jetable selon l'une quelconque des revendications 1 à 7, dans lequel le récipient (1) a des moyens de fixation à des parties du corps ou d'objets, comme une bande auto-agrippante, un adhésif, des sangles, des rubans et des ceintures, entre autres ; en ce que le récipient (1) est joint au récipient (1) d'autres dispositifs thermiques autonomes identiques ou similaires, formant un seul corps de plusieurs de ceux-ci ; en ce qu'il comprend en outre un bouclier thermique (10) qui isole du froid et distribue la chaleur générée par le récipient (1) dudit dispositif, qui comprend une ou plusieurs couches de matériaux et tissus superposés liés ; en ce qu'un ou plusieurs matériaux des couches dudit bouclier thermique (10) sont choisis dans le groupe constitué de : laine, polyester, matériaux métalliques tels que l'argent ou le cuivre, aérogel, néoprène, ouate de laine, acétate, polyester ou coton, entre autres, et l'un quelconque des mélanges de ceux-ci, de préférence trois couches de néoprène, aérogel et laine ; en ce qu'en ce qu'un ou plusieurs matériaux métalliques de l'une quelconque des couches du bouclier thermique (10) sont l'argent ou le cuivre, en pourcentages de 5 à 100 %, sur une ou les deux faces ; en ce qu'une ou plusieurs couches superposées du bouclier thermique (10) sont jointes par un quelconque système de jonction, choisi dans le groupe constitué de : couture, boutons, fermoirs, fermeture à glissière, bande auto-aggripante, lacets et œillets, goujons, colle, spray de thermofusion, colle, thermoscellage, entre autres, en fonction de l'objet ou du vêtement sur lequel **il** est adhéré ; en ce que ledit bouclier thermique (10) est incorporé et accouplé à un quelconque objet non textile ou textile, un article d'habillement pour personnes ou animaux déjà commercialisé, situé sur ces derniers dans les zones proches du dispositif thermique, recouvrant ou situé sur le récipient (1) ; et en ce que le bouclier thermique (10) est fabriqué sous la forme d'objets ou d'articles d'habillement.

9. Dispositif thermique autonome jetable selon l'une quelconque des revendications 1 à 8, comprenant en outre un couvercle thermique (31) qui distribue la chaleur générée par le dispositif thermique à un quelconque objet ou des parties de celui-ci, des surfaces, des êtres vivants, des aliments ou des boissons, qui comprend une ou plusieurs feuilles isolantes (34) entre lesquelles un creux (33) est défini qui loge le récipient (1) du dispositif et un système de fermeture ; en ce que lesdites une ou plusieurs feuilles (34) sont faites d'un ou plusieurs types de matériaux choisis dans le groupe comprenant les polymères d'origine inorganique, organique naturelle et organique synthétique ; en ce qu'une ou plusieurs feuilles (34) sont faites de matériaux choisis dans le groupe constitué par le polyuréthane ou le polyester ou le PVC ou la mousse de polyéthylène ; dans ses mousses ou agglomérés ou extrudés ou dans les mousses à mémoire de forme ; des élastomères tels que le néoprène, le lycra ou le spandex ; le liège, le carton, le caoutchouc, l'aérogel, ou ils sont constitués d'un tissu ou d'une maille (35) qui incorpore des matériaux métalliques tels que l'argent ou le cuivre, en pourcentages de 5 à 100 %, de préférence en pourcentages de ces métaux entre 5 et 40 %, ou des mélanges de ceux-ci ; en ce que le couvercle (31) a des feuilles de mousse de polyuréthane (34), qui sont séparées en laissant un creux (33) qui loge le récipient (1) du dispositif ; étant agencées de telle sorte qu'il y a une feuille de caoutchouc mousse recouverte d'un tissu sur les deux faces, un creux (33) qui loge le récipient (1) du dispositif thermique autonome et une feuille d'aérogel recouverte de tissu sur les deux faces ; en ce que lesdites feuilles de mousse de polyuréthane (34) ont une épaisseur comprise entre 1 mm et 300 mm et une densité comprise entre 5 kg/m3 et 70 kg/m3, de préférence entre 10 kg/m3 et 30 kg/m3 ; et en ce que l'une ou les deux feuilles de mousse de polyuréthane (34) sont du type souple très flexible.

10. Dispositif thermique autonome jetable selon la revendication 9, dans lequel le couvercle (31) est recouvert à l'intérieur, à l'extérieur ou les deux par un tissu ou une maille (35) dans lequel un ou plusieurs matériaux dudit tissu ou de ladite maille (35) sont choisis dans le groupe constitué par un quelconque matériau textile naturel tel que le coton, la laine, la soie, le cuir, la micro-laine et le lin, entre autres, ou des matières synthétiques telles que le polyamide, le nylon, le polyester, le PVC, le trilaminé, le tétralaminate, le vinyle, le néoprène, le PU, le lycra ou le spandex, des tissus métalliques cuivrés ou argentés, la silicone, le latex, le néoprène, du tissu anti-acariens, le polyester, l'aérogel, le tissu non tissé, le plastique aluminisé ou non, le nylon, le vinyle, entre autres, ou l'un quelconque des mélanges de ceux-ci ; en ce que la couverture (31) contient, sur ledit tissu ou ladite maille (35), un revêtement en néoprène supplémentaire ; en ce que le tissu ou la maille (35) contient des matériaux métalliques tels que l'argent ou le cuivre, en pourcentages de 5 et 40 % ; en ce que le couvercle (31) comprend une fermeture à glissière (32), une bande auto-aggripante (36), un rabat avec bande auto-aggripante (37), des aimants, des fermoirs, des boutons-pression ou autres pour isoler l'intérieur du dispositif thermique ; en ce qu'il comprend des systèmes de fixation pour se fixer ou s'adapter au corps de personnes, d'animaux, des articles d'habillement, de la literie, des sacs de couchage, des harnais et un quelconque objet à chauffer, choisis dans le groupe constitué par des sangles, des rubans, des ceintures, une bande auto-aggripante, de la colle, un adhésif, ou une feuille magnétique (39) ou des aimants (40), ou avec des boucles, une couture, etc ; et en ce que le couvercle (31) a une configuration qui comprend une ou plusieurs languettes dans la position supérieure (41), inférieure (42) et latérale (43), avec des systèmes de fermeture, qui servent à adhérer et à s'ajuster à des objets tels que les sangles (219) d'un harnais.

11. Dispositif thermique autonome jetable selon l'une quelconque des revendications 9 ou 10, dans lequel le couvercle (31) comprend une poche antiradiation (38), pour éviter la radiation de dispositifs mobiles et le vol des données de ceux-ci ou de cartes bancaires, etc. ; en ce que le couvercle (31) contient un bouclier thermique (10) tel que décrit dans les revendications 39 à 44 ; en ce que le couvercle (31) contient un indicateur de température (44) ; en ce que le couvercle (31) contient un ou plusieurs générateurs électriques photovoltaïques (45) pour générer et alimenter de l'énergie électrique pour charger une quelconque batterie ou directement un quelconque dispositif mobile électronique tel que les téléphones mobiles, des ordinateurs ou des tablettes entre autres, ou un quelconque appareil électrique ou électronique ; en ce que le couvercle (31) contient un ou plusieurs générateurs thermoélectriques (46) qui utilisent la chaleur générée par le dispositif thermique, dont le récipient (1) est incorporé dans ledit couvercle (31) pour générer et alimenter de l'énergie électrique pour charger une quelconque batterie ou directement un quelconque dispositif mobile électronique tel que les téléphones mobiles, des ordinateurs ou des tablettes entre autres, ou un quelconque appareil électrique ou électronique ; et en ce que le couvercle (31) contient une ou plusieurs bornes pour charger des dispositifs électroniques et/ou une ou plusieurs batteries.

12. Dispositif thermique autonome jetable selon l'une quelconque des revendications 9 à 11, dans lequel le couvercle (31) contient une enveloppe qui comprend, ou qui peut être incorporée à l'intérieur de celui-ci, des composés pharmaceutiques, des insecticides, des huiles essentielles et des composés aromatiques non actifs et actifs, des extraits et des composés de plantes naturels tels que les huiles de thé, l'huile de neem, l'huile essentielle d'eucalyptus citronné, l'huile de citronnelle, la sarriette, l'ail, le basilic, la bergamote, la cannelle, la cardamome, l'oignon, le cèdre, la coriandre, la citronnelle, le clou de girofle, le genévrier, l'estragon, l'eucalyptus, le géranium, le lemongrass, le fenouil, la feuille de laurier, la lavande, le citron, la marjolaine, la mélisse, la menthe, la moutarde, l'orange, l'origan, le bois de rose, le paprika, le persil, le pin, le pamplemousse, le romarin, la sauge, le soja, le thym, la vanille, etc. ou des mélanges de ceux-ci ; en ce qu'il comprend un activateur (50) applicable au couvercle (31) qui comprend un capot (47) avec une ou plusieurs gâches (48) et une base (49), les deux joints sur l'extérieur des faces opposées du couvercle thermique (31), de telle sorte que, lorsque le capot (47) est pressé, la gâche ou les gâches (48) passent à travers le couvercle thermique (31), la feuille (5) du récipient (1) du dispositif thermique et le récipient secondaire (7), et le liquide aqueux (6) sort en entrant en contact avec les réactifs de la composition exothermique (4) activant la réaction exothermique ; en ce que le couvercle (31) est fabriqué sous la forme d'une quelconque partie du corps de personnes ou d'animaux afin d'y être directement accouplé ; et en ce que le couvercle (31) est fabriqué sous la forme d'objets ou d'articles d'habillement ou pour être incorporé et accouplé à un quelconque objet non textile ou textile, un article d'habillement pour les personnes ou les animaux.

13. Dispositif thermique autonome jetable selon l'une quelconque des revendications 9 à 12, dans lequel le récipient (1) est incorporé, logé dans le couvercle (31) comportant ou non le bouclier thermique (10), aux sangles (219) d'un harnais pour le fixer au corps d'un utilisateur ; et en ce que les deux sangles (219) du harnais et/ou un ou plusieurs couvercles thermiques (31) incorporés dans ledit harnais contiennent des accessoires tels que des anneaux porte-matériaux (223), des protections rembourrées (224), des poches, des mousquetons et des goupilles.

14. Objet comprenant un dispositif thermique autonome jetable selon l'une quelconque des revendications précédentes.

15. Objet selon la revendication 14, constitué par une serpillière thermique qui comprend un corps (133) pourvu d'un creux pour loger le récipient (1) du dispositif avec un capot de fermeture (138) ; en ce que le matériau du corps et du couvercle de ladite serpillière thermique est choisi dans le groupe constitué par l'or, l'argent, le platine, le cuivre, l'aluminium, le fer, le plomb, l'acier, l'étain, le zinc, le nickel, le magnésium, le manganèse, le titane, le béryllium ou l'un quelconque des alliages de ceux-ci, des céramiques ou des composites à matrice céramique à haute conductivité thermique, du carbone ou du graphite ou un quelconque type de plastique ; en ce que, de plus, **il** incorpore une ou plusieurs feuilles faites de matériaux isolants, tels que la mousse de polyuréthane, sur la base des faces internes du corps (133) et du capot (139) ; en ce qu'il comprend un ou plusieurs couvercles thermiques (31) faisant partie du corps de la serpillière thermique ; en ce qu'il comprend un bouclier thermique (10) formant une partie du corps de la serpillière thermique ; en ce que le corps (133) de la serpillière contient des systèmes de fixation du récipient (1) du dispositif thermique autonome, tels qu'un cadre indépendant avec des languettes (137) sur le bord inférieur du corps ; en ce que le corps (133) est ajusté au capot (138) par le biais d'un système de fermeture à pression, une bande auto-aggripante, des boutons-pression, un levier avec ou sans cadenas ou autre ; en ce que la serpillière contient un robot motorisé automatisé avec des roues ; et en ce que la serpillière contient un indicateur de température avec une alarme acoustique.
